# EUROPEAN PATENT SPECIFICATION

(11) **EP 0 462 145 B1**
(45) Date of publication and mention of the grant of the patent: **27.04.1994**
(21) Application number: 90904028.9
(22) Date of filing: 23.02.1990
(51) Int. Cl.: A61K 31/70, A61K 47/48

(54) **COVALENT CONJUGATES OF LIPID AND OLIGONUCLEOTIDE**
KOVALENTE KONJUGATE VON LIPIDEN UND OLIGONUKLEOTIDEN
CONJUGUE COVALENT DE LIPIDES ET D'OLIGONUCLEOTIDES

(30) Priority: 07.03.1989 US 320202
(43) Date of publication of application: 27.12.1991
(73) Proprietor: GENENTECH, INC., South San Francisco California 94080 (US)
(72) Inventor: BISCHOFBERGER, Norbert, W., San Carlos, CA 94070 (US); SHEA, Regan, G., Pacifica, CA 94044 (US)
(74) Representative: Stuart, Ian Alexander
(86) International application number: US9001002
(87) International publication number: WO9010448

(56) References cited:
- DE-A- 3 637 243
- Chemical Reviews, vol.90, no. 4, June 1990, Am. Chemical Soc., E. Uhlmann et al., pp. 556-562
- STN File Server (Karlsruhe) & File Medline, H. Yanagawa et al., accession no. 89144950
- Nucleic Acids Research, vol. 15, no. 14, 1987, IRL Press Ltd, (Oxford, GB), C.J. Marcus-Sekura et al., pp. 5749-5763
- Biotechniques, vol. 6, no. 10, 1988, A.R. vander Krol et al., pp. 958-974
- STN File Server (Karlsruhe) & File CA, Chemical Abstracts, vol. 109, 1988, (Columbus, Ohio, US), E.E. Bichenkov et al., abstract 225156z
- Proc. Natl. Acad. Sci. USA, vol. 86, Sept. 1989, R.L. Letsinger et al., pp. 6553-6556

## Description

This invention relates to covalent conjugates of lipids and oligonucleotides and their use in the pharmaceutical, purification, and diagnostic arts.

Use of antisense RNA or DNA has proven useful for manipulating eukaryotic gene expression. This technique is based on blocking the informational flow from DNA to protein via RNA by introducing a complementary sequence to a portion of the target mRNA in the cell. When the oligomer is absorbed by the cells, it can either function in the cytoplasm by blocking translation of the target messenger or, upon penetrating the nucleus, interfere with nuclear processing or transcription by binding to DNA. In the case of viral infection, antisense oligomers may also function by blocking viral replication, depending on the target site of the oligomer on the viral genome. Complementary nucleic acids that inactivate gene expression have been designated antisense nucleic acids.

Efficient transport of nucleic acids across cell membranes is of great importance for antisense nucleic acid technology, as well as in simple transfection experiments. For recent reviews of antisense oligonucleotides, see van der Krol *et al*., BioTechniques, 6: 958 (1988) and Zon, Pharmaceutical Res., 5: 539 (1988).

Uptake of nucleotides by cells and liposomes is normally an inefficient process due to the high charge density of the nucleotide. A number of schemes have been used to improve incorporation efficiency, namely, calcium phosphate-mediated gene transfer (Chen, C.A. and Okayama, H., BioTechniques, 6:632 (1988)), use of retroviral vectors (Eglitis, M.A.*et al*., BioTechniques, 6: 608 (1988)), microinjection (DePamphilis, M.L. *et al*., BioTechniques, 6: 662 (1988)), use of adenovirus vectors (Berkner, K.L., BioTechniques, 6: 616 (1988)), electroporation (Andreason, G.L. and Evans, G.A., BioTechniques, 6: 650 (1988)), rapid acceleration of DNA-coated, gold particles (Christou P. *et al*., Plant Physiol., 87: 671-674 (1988)), and liposome-mediated gene transfer (Mannino, R.J. and Gould-Fogerite, S., BioTechniques, 6: 682 (1988)). All of these methods, however, suffer from one or more problems relating to cellular toxicity, introduction of potentially pathogenic viral factors, poor reproducibility, inconvenience, or inefficiency of DNA delivery.

More recently, DNA covalently attached to cell receptor ligand proteins, such as that for epidermal growth factor, was studied as a means to effect cell-specific DNA delivery. EP 273,085 published July 6, 1988. Additionally, DNAs complexed with sugar-lysine conjugates (Wu, G.Y. and Wu, C.H., J. Biol. Chem., 263: 14621 (1988)) and with a positively charged lipid, i.e., N-[1-(2,3-dioleyloxy)propyl]-N,N,N-trimethylammonium chloride, entrapped in a liposome (Felgner, P.L. et al., Proc. Natl. Acad. Sci. USA, 84: 7413 (1987)), were shown to enhance transfection efficiency.

Protein-DNA conjugates are found in nature and are important in viral replication. Vartapetian and Bogdanov, A.A. Prog. Nucl. Acid Res. Mol. Biol., 34: 210 (1987). Non-natural hybrids have been prepared in which DNA is linked to targeting, cleaving, or reporter groups, including peptides, biotin, fluorescent dyes, and EDTA-Fe. Zuckermann et al., J. Am. Chem. Soc., 110: 6592 (1988); Haralambidis et al., Tetrahedron Lett., 28: 5199 (1987); Connolly, Nucl. Acids Res., 15: 3131 (1987); Agrawal et al., Nucl. Acids Res., 14: 6227 (1986); Smith et al., Nucl. Acids Res., 13: 2399 (1985); Moser and Dervan, Science, 238: 645 (1987).

Phosphatidyl nucleosides are biosynthetic intermediates in the lipid metabolism and a number of derivatives thereof showed interesting biological activities. Shuto, S. *et al*., Chem. Pharm. Bull., 36: 209 (1988) (5'-(3-sn-phosphatidyl)nucleosides for antileukemic activity); U.S. Pat. No. 4,797,479 issued Jan. 10, 1989 to Shuto et al. (phospholipidnucleoside complex prepared by reacting L-glycerophospholipid with nucleoside in the presence of phospholipase D for treatment of tumors); Matsushita, T. *et al*., Cancer Res., 41: 2707 (1981) (nucleoside 5'-diphosphate-L-1,2-dipalmitin derivatives of 1-β-D-arabinofuranosylcytosine, 9-β-D-arabinofuranosyladenine, and tubercidin for enhanced catabolic stability); Turcotte, J.G., *et al*., Biochem. Biophys. Acta, 619: 604 (1980) and 619: 619 (1980) (analogs of cytidine diphosphate diacylglycerol containing the 1-β-D-arabinofuranosyl moiety for anticancer activity). A cholesterol-nucleoside conjugate was recently reported for application in liposomes. Hashida *et al*., Chem. Pharm. Bull., 36: 3186 (1988).

Combinations of ether lipid analogs and DNA-interactive agents, i.e., adriamycin, 4-hydroperoxycyclophosphamide, and cisplatin, were found to enhance anti-tumor activity in an additive fashion. Noseda *et al.*, Cancer Res., 48: 1788-1791 (1988).

Transfection methods applicable for *in vivo* therapeutic purposes, e.g., in which foreign sequences of active nucleic acids can be applied to a living organism to cure some deficiencies of the cell metabolism, have been developed. For example, Cheng et al., Nucl. Acids Res., 11: 654-669 (1983) reports that the construct of chloramphenicolacetyltransferase gene bound to a 2-macroglobulin was internalized in 3T3-4 cells. However, no evidence was provided that the internalized DNA was capable of performing a function in the host cells. EP 273,085, published July 6, 1988, discloses linking a nucleic acid to a cell homing or targeting factor that promotes the penetration of the foreign nucleotides through the cell membrane, but has little specificity regarding cell recognition. Such targeting factors include low density lipoproteins, growth factors, viral antigens, and the B chain of toxins.

The use of hydrophobic 5'-protecting groups for the HPLC purification of chemically synthesized oligodeoxynucleotides by solid support methods was recently investigated. Seliger and Schmidt, J. Chromatography 397: 141-151 (1987); Schmidt et al., Nucleosides and Nucleotides, 7: 795-799 (1988).

It is an object of the present invention to provide a vehicle for introducing DNA across cell membranes that is highly efficient.

It is another object to provide a transfection technique for internalization of active oligo- and polynucleotides, e.g., antisense DNA that can hybridize to mRNA in the cell and thus inhibit cellular or viral functions.

It is yet another object to provide an agent that is more stable than DNA-lipid complexes and is a substrate for cellular lipases so that the molecule will be cleaved when it comes into contact with membrane-bound intracellular cytoplasmic enzymes to free the drug for use by the cell.

It is a further object to provide a liposome encapsulating such an agent.

It is still a further object to provide an assay for nucleic acids and antibodies incorporating the agent.

These and other objects will be apparent to one of ordinary skill in the art. According to the present invention there is provided a covalent conjugate of a lipid and an oligonucleotide, or a pharmaceutically acceptable salt thereof, wherein the lipid is covalently linked through phosphate to the 5'- end of the oligonucleotide, and is selected from steroids, glycerides and glyceryl ethers; with the proviso that the conjugate is not nonathymidyluridine modified at the 5'-phosphate with cholesterol. Preferably, the oligonucleotide has a nucleotide sequence sufficiently complementary to a pathogenic nucleic acid or an oncogene to hybridize thereto.

The conjugate may be labeled or immobilized on a solid support.

The above conjugate may be introduced into a host cell by transfecting the host cell with the conjugate.

The conjugate may be formulated as a composition comprising a pharmaceutically acceptable carrier and the conjugate, with the carrier preferably being a liposome. This may be used in a method comprising administering to a plant, animal, or human suffering from a pathogenic condition an effective amount of the above composition.

In still another aspect, the invention provides a method for the assay of a nucleic acid having a predetermined nucleotide sequence in a sample comprising:
(a) providing the conjugate according to the first aspect of the invention as a labeled conjugate that has a nucleotide sequence capable of hybridizing to the predetermined sequence;
(b) immobilizing the labeled conjugate on a support;
(c) contacting the sample with the immobilized conjugate under conditions that would cause hybridization of the nucleic acid with the oligonucleotide portion of the conjugate if the nucleic acid is present in the sample; and
(d) detecting the presence of labeled oligomers.

In an additional aspect, the invention provides a method for separating an oligonucleotide from a mixture, which method comprises:
(a) providing the conjugate having a nucleotide sequence capable of hybridizing to a sequence contained within the oligonucleotide to be separated;
(b) immobilizing the conjugate on a support;
(c) contacting the mixture with the immobilized conjugate, under conditions causing hybridization of the oligonucleotide of the mixture with the oligonucleotide portion of the conjugate; and
(d) separating the hybridized oligonucleotides.

In yet another aspect, the invention provides a method for separating an oligonucleotide from a mixture, which method comprises:
(a) providing the conjugate according to the first aspect have a nucleotide sequence capable of hybridizing to a sequence contained within the oligonucleotide to be separated in a hydrophobic phase;
(b) providing the mixture in a hydrophilic phase;
(c) contacting the phase containing the mixture with the phase containing the conjugate, under conditions causing hybridization of the otigonucleotide of the mixture with the oligonucleotide portion of the conjugate; and
(d) extracting from the phase containing the mixture the hybridized otigonucleotides and transporting them to a separate hydrophiIic phase.

In another aspect, the invention provides a method for detecting lupus erythematosus in a human comprising contacting a serum sample from the human with a liposome comprising the conjugate and a label, said contacting being such that the antibodies bind to the oligonucleotide on the liposome, so as to alter the stability of the liposome; and measuring for the presence or absence of the label.

The invention herein revolves around covalent conjugates of lipids and oligonucleotides that contain nucleotide sequences of interest or are sufficiently complementary to hybridize to sequences of interest. The lipid may act to internalize the nucleic acid sequence chemically coupled thereto into host cells, typically by endocytosis, and is bound to the oligonucleotide through a coupling sufficiently labile that it does not repress subsequent biochemical functioning of the oligonucleotide in the cell after internalization. In fact, preferably the conjugate is a substrate for a cellular enzyme that cleaves the conjugate to release the oligonucleotide. The lipid is suitably covalently bonded to any moiety on the oligonucleotide, such as an amino group on the base, the hydroxyl group of the phosphate, or a hydroxyl group at the 5' or 3' terminus of the oligonucleotide. Preferably, however, it is bonded to the 5' hydroxyl group thereof.

Of particular interest herein are conjugates containing a cleavage site within the lipid or representing the covalent bond between the lipid and oligonucleotide that is specifically recognized by an enzyme endogenous to a host cell to which the conjugate is targeted. Thus, cleavage at the site will break the bond or hydrolyze the lipid so as to render the conjugate more water soluble. Preferably the cleavage site is susceptible to enzyme hydrolysis. Also preferred is that the enzyme is located at a cellular or nuclear membrane or is in the cytoplasm of a cell.

The most preferred lipids herein are phospholipids, where the enzyme that recognizes the cleavage site is a lipase, preferably a phospholipase. Also, preferably, the cleavage site is the bond between the 5'-hydroxyl oxygen atom of the oligonucleotide and the phosphorus atom (in the case of phospholipase D) or the bond between the oxygen atom on the glycerol side and the phosphorus atom (in the case of phospholipase C). Phospholipase C and D are ubiquitous membrane-bound cytoplasmic enzymes that catalyze the hydrolysis of certain phosphoester bonds, thereby cleaving the conjugate and releasing the oligonucleotide into the cytoplasm. For a review of phospholipases, see Dennis, E.A. in "The Enzymes," Vol. XVI (New York: Academic Press, Inc., 1983), p. 307-353). It is preferred to maximize cleavage by phospholipase C or D rather than other lipases that cleave fatty acids so as to leave lipid residue on the oligonucleotide.

Suitable phospholipids herein include, e.g., phosphoglycerides, plasmalogens, and other phosphatidic acids, sphingomyelin, and 3'-O-aminoacyl phosphatidyl glycerol.

The "oligonucleotide" portion of the conjugate refers to a molecule comprised of two or more deoxyribonucleotides or ribonucleotides that has a nucleotide sequence that is either of interest or sufficiently complementary to hybridize to a nucleotide sequence of interest. The oligonucleotide is typically one that is capable of performing a biochemical function in receptor host cells and/or altering the operation of the cell machinery. Its exact size will depend on many factors, which in turn depend on the ultimate function or use of the oligonucleotide. For example, if the oligonucleotide is to be used as an antisense oligomer, to ensure specificity thereof, the sequence must be unique in the mRNA population, and complementary to the target RNA.

It has been calculated that a chain length of 14 nucleotides may be sufficient to define the sequence specificity of oligonucleotides. See Ts'O, *et al.*, "Biological Approaches to the Controlled Delivery of Drugs" Vol. 507, Ann. N.Y.Acad. Sciences, 1987. Increasing the length of the oligonucleotide increases stability of the duplex formed and thus its inhibitory effect. On the other hand, very long oligomers can interact with two or more mRNAs through base pairing involving only 5-10 contiguous bases within the sequence of the oligonucleotide, thus lowering the specificity of the oligonucleotide. For general use, the oligonucleotide contains at least five bases, more preferably, from about five to about thirty bases, and most preferably from about fourteen to about twenty-five bases.

The oligonucleotides herein are selected either to contain, or to be sufficiently complementary to hybridize to, the nucleotide sequence of interest. Therefore, the oligonucleotide sequence need not reflect the exact sequence of the nucleotide sequence of interest. For example, a non-complementary nucleotide fragment may be attached to the 5' end of the oligonucleotide, with the remainder of the sequence being complementary to the nucleotide strand of interest. Alternatively, non-complementary bases or longer sequences can be interspersed into the oligonucleotide, provided that its sequence be capable of hybridizing to the target nucleic acid.

For purposes herein, the term "oligonucleotide" also includes oligonucleotides having modifications of the sugar-phosphodiester backbone to reduce their sensitivity to cellular nucleases and increase absorption by the cell as needed. One example is methylphosphonates wherein one of the oxygen atoms is substituted by a methyl group to result in four different substituents on the phosphorus atom, as disclosed by van der Krol *et al.*, *supra* in Table 1. Oligomer methylphosphonates directed against HSV-1 immediate early mRNA-4 and -5 were found to prevent expression of herpetic lesions when applied in the form of cream to the HSV-infected ear of a mouse and were not toxic to mice when injected intravenously in concentrations up to 40 mg/kg body weight. Miller *et al*., Anti-Cancer Drug Design, 2: 117-128 (1987); Ts'O *et al.*, *supra*. Additionally, phosphorothioates have been found to be potent inhibitors of viral proliferation, particularly HIV. Matsukura *et al*., Proc. Natl. Acad. Sci., U.S.A., 84: 7706-7710 (1987); Agrawal *et al*., Proc. Natl. Acad. Sci. U.S.A., 85: 7079-7083 (1988).

The definition of "oligonucleotides" also includes those with a covalently linked reagent besides the lipid that increases affinity for the complementary sequence. For example, coupling of poly-(L-lysine) to the 3' end of an antisense VSV oligonucleotide may be employed to enhance its antiviral activity. Further, a phosphorothioate oligomer may be coupled to a poly-(L-lysine) to lower the effective dose. In addition, a phosphorothioate oligonucleotide is useful herein because it is stable to cleavage by nucleases, is very soluble in water, and hybridizes more efficiently with a complementary DNA sequence that the corresponding methylphosphonate analogs. See, e.g., Marcus-Sekura et al., Nucl. Acids Res., 15: 5749-5763 (1987). An intercalating agent such as acridine may be added to the 3' end of the oligonucleotide to enhance its affinity for the target. Other methods include attaching to the antisense oligomer a reactive agent to modify the target nucleic acid irreversibly, including alkylating reagents, metal complexes such as EDTA-Fe(II), o-phenanthroline-Cu(I), or porphyrin-Fe(II). Such compounds generate hydroxyl radicals in the presence of molecular oxygen and a reducing agent and cleave the complementary strand following attack on the target nucleic acid backbone. In addition, a photocrosslinking agent can be attached to the oligomer, such as a psoralen derivative, azidophenacyl, and proflavine.

Preferably the lipid is a glyceride or glyceryl ether of the formula 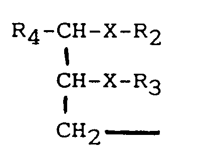
wherein R₂, R₃ and R₄ are independently selected from H, C₁-C₃₀ alkyl, C₂-C₃₀ mono-, di-, or polyunsaturated alkyl, C₃-C₈ cycloalkyl or C₄-C₈ mono-, di-, or polyunsaturated cycloalkyl group and X is 0 or -0.CO-.

Preferably the oligonucleotide moiety is selected from 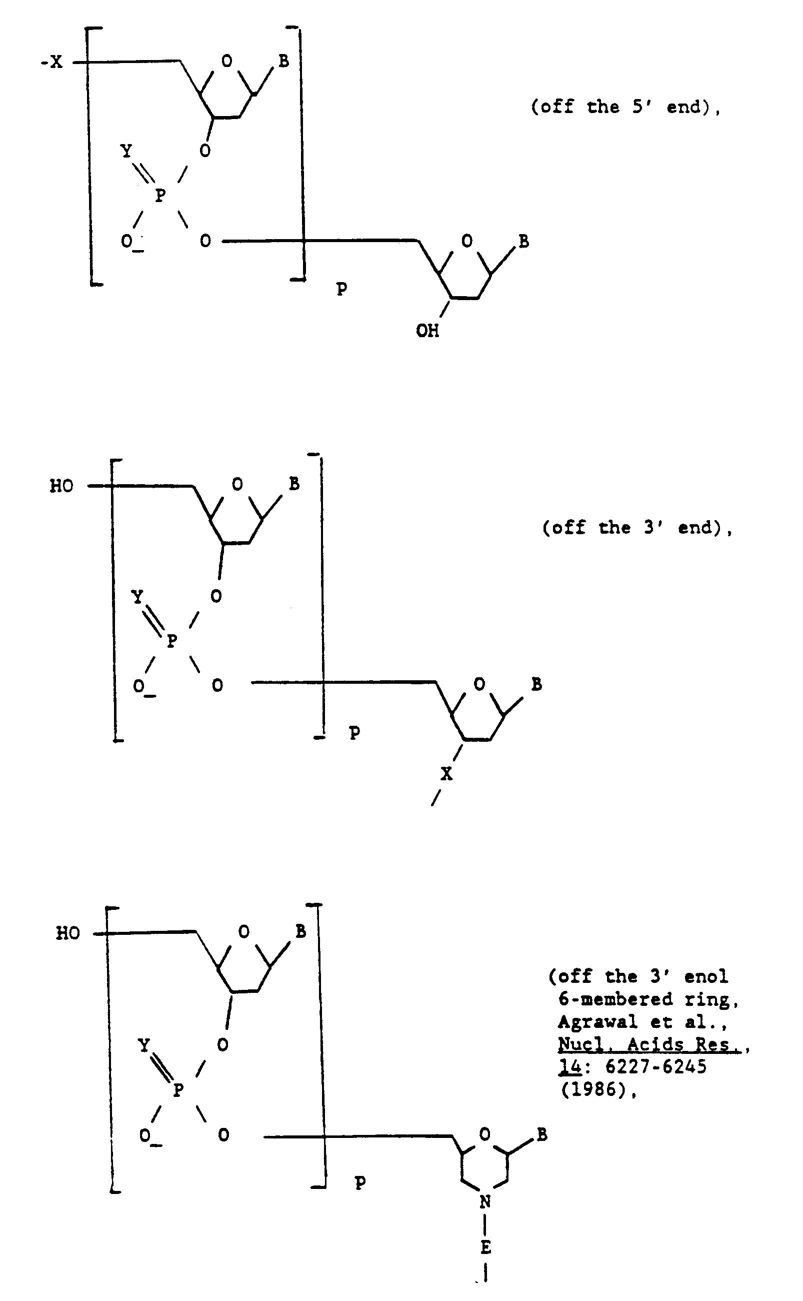 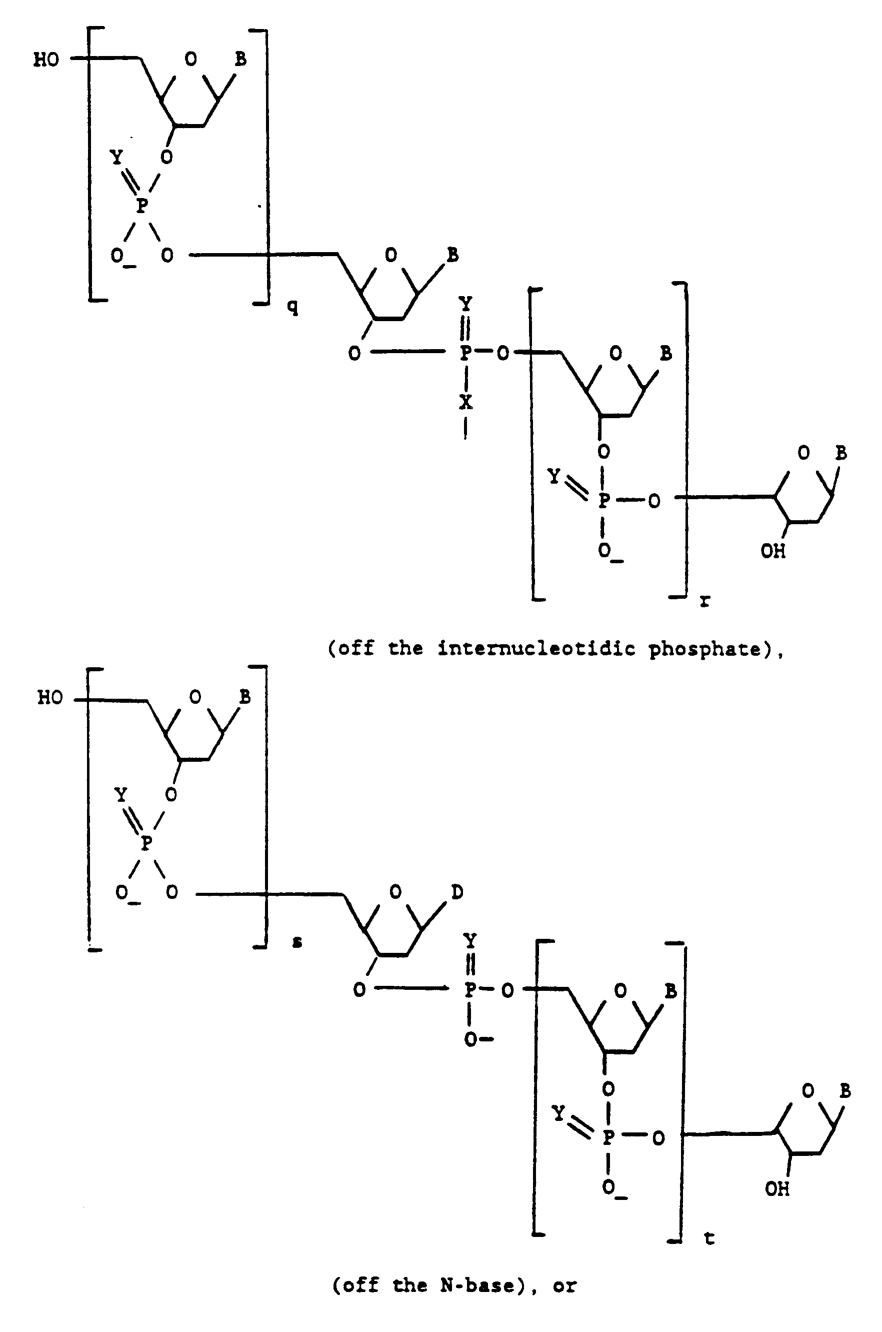 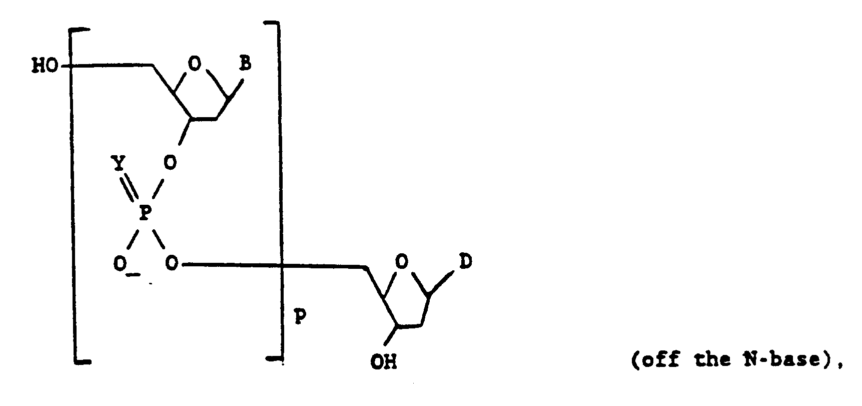
where B is a deprotected base, p is an integer from about 5 to 30, q and r are integers from about 1 to 28, provided that r + q is from about 4 to 29, s and t are integers from 0 to about 29, provided that s + t is from about 4 to 29, E is X or ZX, and D is selected from the group consisting of (where the bond from the ring nitrogens is attached to the sugar moiety of the oligonucleotide):

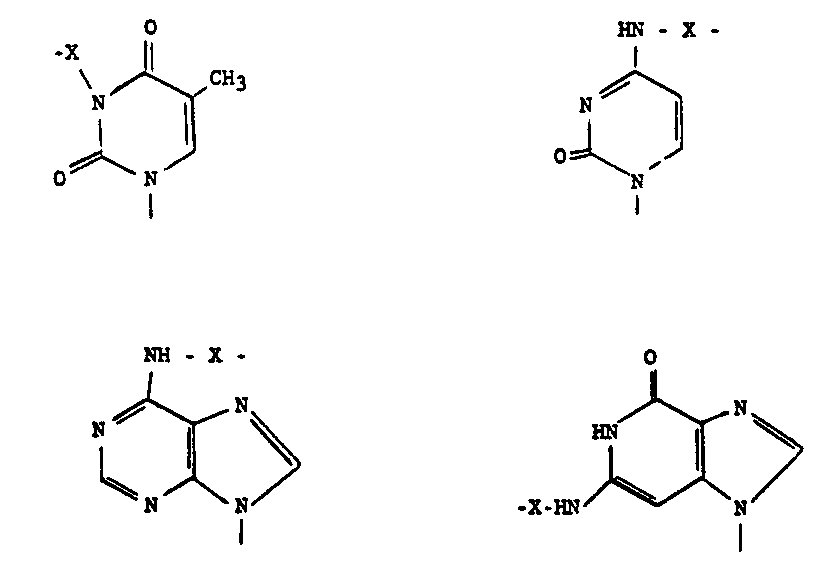

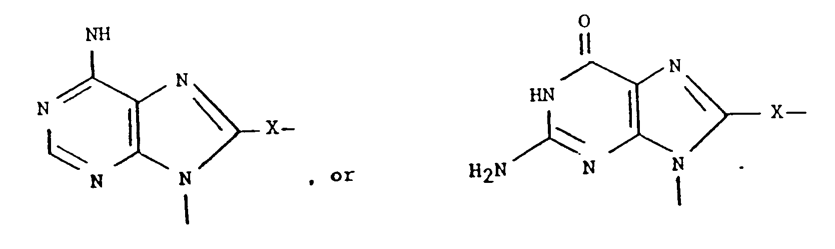

The terms "alkyl" and "cycloalkyl" as used herein refer to both straight-chain and branched alkyl groups ad cycloalkyl groups with straight-chain or branched alkyl groups pending from the ring. "Mono-, di- and polyunsaturated" alkyl, cycloalkyl, or alkylene (divalent alkyl) moieties refer to hydrocarbons that contain one or more carbon-carbon double or triple bonds anywhere along their chain. Examples of compounds that may provide unsaturated cyclic or acyclic moieties include terpenes, such as phytol, geraniol, limonene, farnesol, and squalene, vitamins, such as vitamins A, D, and K, and β-carotene, and prostaglandins.

If the lipid is a steroid moiety, preferably it is a sterol, and more preferably a sterol linked to the oligonucleotide through the hydroxyl group (either as an ether or ester) at its C₃ position. Examples of suitable steroids include cholesterol, lanosterol, a phytosterol, or a mycosterol such as, e.g., ergosterol.

If the oligonucleotide is connected off the N-base, it is preferably off the N6 of an adenine residue or the N4 of a cytosine residue.

If it is desired to provide oligonucleotides containing intercalating bases that are sterically confined so as to inactivate translationally the targeted strand, at least one of the bases is a substantially planar base. The oligonucleotides so derivatized hybridize to their complementary RNA or DNA strand, the modified nucleoside remaining unpaired but nonetheless intercalated between adjacent base pairs in the duplex in a precisely sterochemically defined manner. In light of the precise steric targeting made possible by this invention, the intercalating moiety may be substituted with a reactive group capable of covalently modifying a predetermined site in the complementary domain. Such reactive groups include crosslinking agents and phosphate bond cleaving agents. These reactive groups are sterically confined and less likely to interact with cellular components or nucleic acid at sites other than the target complementary sequence.

The phrase "substantially planar" in this context means that the steric bulk of the group lies substantially within an envelope approximating the steric gap bounded by the sugar backbone and flanking bases present in a complementary nucleic acid strand. In general, this envelope has dimensions of about 30-50 Angstroms in width and depth and about 3-7 Angstroms in thickness. An example includes a nucleoside having the structure: 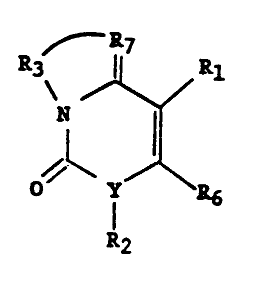
wherein R₃ is an aromatic polycycle, Y is C or N, R₇ is N or =C(R₁)-, R₁ and R₆ are H or a halogen, nitro, alkyl, hydroxyalkyl, or alkylether group wherein the alkyl group has 1 to 10 carbon atoms, and R₂ is a ribose or deoxyribose sugar.

The nucleic acid sequences of interest include mRNA and DNA, and may be present in restriction enzyme digests or other fragments of nucleic acids, e.g., restriction fragment length polymorphisms (RFLPs). The nucleic acid or acids may be obtained from any source, for example, from plasmids such as pBR322, from cloned DNA or RNA, or from natural DNA or RNA from any source, including bacteria, yeast, viruses, and higher organisms such as plants or animals. For therapeutic purposes, the antisense oligonucleotides should be selected so as to have an efficient and specific interaction with the target mRNA, an efficient cellular uptake and compartmentalization of the oligonucleotide, and sufficient stability in the different cellular compartments.

For diagnostic use, the oligonucleotide may contain a sequence that encodes a diagnostically useful protein, e.g., pathogenic proteins, such as those responsible for viral infections, including AIDS, oncogenes, growth factors, β-globin, and the like. Further, other nucleic acids that encode no protein may be of interest, e.g., transcription or translation control domains or sequences useful in forensic medicine.

Most preferably, the conjugates herein have utility as agents for the antisense inhibition of translation of target nucleic acids. Such utilities have already been explored extensively with other antisense oligonucleotides (see van der Krol *et al*., *supra*, and WO 83/01451 published April 28, 1983), and the oligonucleotides herein will be used in substantially the same fashion, using a rational, specific design based on the sequence and secondary structure information of the target RNA or DNA, keeping in mind that the secondary or tertiary structures in the antisense RNA may influence the extent or rate of hybridization. For this use, the oligonucleotide has a nucleotide sequence capable of hybridizing to a pathogenic nucleic acid or an oncogene, more preferably viral or parasitic nucleic acid infecting a plant, such as a vegetable plant; animal, such as domestic, sports, and farm animals, e. g., dogs, cats, cows, and pigs; or human. For example, antisense oligonucleotides, either modified or unmodified, can be used to inhibit viral infections of Rous sarcoma virus, vesicular stomatitis virus, type A influenza virus, herpes simplex virus, human immunodeficiency virus, and plant viruses such as potato virus X coat protein gene and cucumber mosaic virus coat protein gene, to inhibit parasitic, e.g., malarial, infections, and to reduce expression of proto-oncogene c-mvc (for leukemia treatment), simian sarcoma virus, β-globin, and β-tubulin genes. Examples of published viral sequences that have inhibitory effects include those against Rous sarcoma virus (Zamecnik and Stephenson, Proc. Natl. Acad. Sci. U.S.A., 75: 280-284 (1978); Stephenson and Zamecnik, Proc. Natl. Acad. Sci. U.S.A., 75: 285-288 (1978)), those against human T-cell lymphotropic virus type III (Zamecnik et al., Proc. Natl. Acad. Sci. U.S.A., 83: 4143-4146 (1986); WO 87/07300 published Dec. 3, 1987), and those against herpes simplex virus type 1 (Smith et al., Proc. Natl. Acad. Sci. U.S.A., 83: 2787-2791 (1986)).

Included within the definition of useful oligonucleotides for inactivating pathogenic nucleic acids are oligonucleoside alkyl- or arylphosphonate analogues complementary to the target pathogenic sequence and including a functional group that reacts with the target nucleic acid to render it inactive or nonfunctional. These derivatives are described in EP 266,099 published May 4, 1988.

In other embodiments, the oligonucleotide has a nucleotide sequence representing, or capable of hybridizing to, a cleavage site specifically recognized by an enzyme endogenous to a host cell to which the conjugate is targeted. For example, the oligonucleotide may encompass a sequence recognized by the RNase-H enzyme. This enzyme cleaves the RNA part of an RNA/DNA hybrid *in vivo*, resulting in subsequent degradation of the mRNA. Thus, the effect of the antisense oligomer can be catalytically enhanced by RNase-H activity.

Since the splice junctions of pre-mRNAs interact with the RNAs of small ribonucleoprotein particles that mediate the splicing process, these regions of the messenger are ideal targets for complementary oligomers. Thus, the invention herein includes antisense DNA or their analogs directed against splice-junction sites, especially when such antisense DNA has been found to be effective in inhibiting proliferation of HIV (Agrawal *et al*., Proc. Natl. Acad. Sci. U.S.A., 85: 7079-7083 (1988)), HSV (Smith *et al*., Proc. Natl. Acad. Sci. U.S.A., 83: 2787-2791 (1986)), or synthesis of SV 40 large T antigen (Verspieren *et al*., Gene, 61: 307-315 (1987)). In addition, the invention is directed to complementary oligomers that are designed to interact with the cap site or the initiation codon region on the mRNA. Also, it has been shown that antisense oligomers blocking the ribosome attachment site are very effective for this purpose.

The oligonucleotides herein are suitably prepared using conventional methods such as, for example, the phosphotriester or phosphodiester methods described by Narang, S.A. *et al.*, Meth. Enzymol., 68: 90 (1979) and Brown, E.L. *et al*., Meth. Enzymol., 68: 109 (1979), respectively, or automated embodiments thereof. In one such automated embodiment, phosphoramidites are used as starting materials and may be synthesized as described by Beaucage *et al*., Tetrahedron Letters, 22: 1859-1862 (1981). One method for synthesizing oligonucleotides on a modified solid support is described in U.S. Pat. No. 4,458,066, issued July 3, 1984. It is also possible to use an oligonucleotide that has been isolated from a biological source, such as a restriction endonuclease digest.

The conjugates are prepared by any suitable technique. For example, H-phosphonate methodology may be used to synthesize phospholipid conjugates, as described by Lindh and Stawinki, J. Nucleic Acids Res., Symp. Ser. No. 18, 189 (1987). Synthesis of the same compounds is also accomplished using phosphoramidite chemistry as described in Beaucage and Caruthers, Tetrahedron Lett., 1859 (1981) and McBride and Caruthers, Tetrahedron Lett., 245 (1983). The main advantage of using the H-phosphonate methodology is the flexibility in the oxidation procedure. When the oxidation is carried out with carbon tetrachloride in the presence of amines, phosphoroamidates are available, whereas oxidation with sulfur leads to phosphorothioate analogs, and carbon tetrachloride in the presence of alcohols produces phosphate triesters. Froehler, Tetrahedron Lett., 27: 5575 (1986).

Chemical attachment of lipids to oligodeoxynucleotides by the standard solid support DNA synthesis protocol can be employed for lipids that are normally stable to the deblocking conditions, i.e., concentrated ammonia. Such lipids include ether lipids or amide lipids, including triglycerides and sterols that contain amide functionalities in their backbone. Fatty acid derived lipids that contain ester linkages would not normally be stable to such conditions. The latter lipids can be conjugated by using the method of Chu et al., Nucl. Acids. Res., 11: 6513-6529 (1983). This method requires that the lipid contain a free primary or secondary amino group. The lipid may already contain such an amino group in a side chain, as, e.g., in the case of phosphatidyl ethanolamine, phosphatidyl 3'-O-aminoacyl glycerol, and phosphatidyl serine, or it may be derivatized to contain such an amino group. Using the Chu *et al*. method, the aminolipid is coupled to an oligomer-5'-phosphate so as to produce lipid oligonucleotide conjugates. Also, phospholipids may be suitably bonded to oligonucleotides enzymatically using phospholipase D, as has been reported for the synthesis of nucleoside lipid conjugates. See Shuto *et al*., *supra.*

One use for the conjugate herein is for the transfection of host cells so that DNA is stably incorporated therein. The transfection involves merely contacting the cells with the conjugate as by use of stirring at room temperature for at least about 15 hours, preferably about 20-30 hours.

Another use is for the treatment of a mammal with a pathogenic condition that is alleviated or cured by use of an oligonucleotide with a nucleotide sequence that blocks a nucleic acid responsible for the condition. Examples of such conditions include those caused by oncogenes and infections caused by viruses such as AIDS, herpes, hepatitis, etc. The mammal to be treated may be any mammalian species such as domestic and farm animals, including primates, and sports or pet animals, as well as humans. Preferably, however, the preferred species being treated is human.

The conjugate is administered to the patient by any suitable technique, including parenteral, sublingual, topical, intrapulmonary, and intranasal administration. The specific route of administration will depend, e.g., on the type of therapy required. Examples of parenteral administration include intramuscular, subcutaneous, intravenous, intraarterial, and intraperitoneal administration.

The conjugates to be used in the therapy will be formulated and dosed in a fashion consistent with good medical practice taking into account the clinical condition of the, individual patient, the cause of the condition in need of therapy, the site of delivery of the conjugate, the method of administration, the scheduling of administration, and other factors known to practitioners. The "effective amount" for purposes herein is thus determined by such considerations.

As a general proposition, the total pharmaceutically effective amount of the conjugate administered parenterally per dose will be in the range of about 1 µg/kg/day to 100 mg/kg/day of patient body weight, although, as noted above, this will be subject to a great deal of therapeutic discretion. The key factor in selecting an appropriate dose is the result obtained, as measured by elimination of infection or reduction in tumor or by other criteria as deemed appropriate by the practitioner.

For parenteral administration, the conjugate is formulated generally by mixing it at the desired degree of purity, in a unit dosage injectable form (solution, suspension, or emulsion), with a pharmaceutically acceptable carrier, excipient, or stabilizer, i.e., one that is non-toxic to recipients at the dosages and concentrations employed and is compatible with other ingredients of the formulation. Such materials include hydrophobic polymers, monosaccharides, disaccharides, and other carbohydrates including cellulose or its derivatives, glucose, mannose, or dextrins, sugar alcohols such as mannitol or sorbitol; and/or nonionic surfactants such as Tween, Pluronics, or PEG.

Generally, the formulations are prepared by contacting the conjugates uniformly and intimately with liquid carriers, homogeneous phospholipid mixtures (for liposome encapsulation), or finely divided solid carriers, and then, if necessary, shaping the product into the desired formulation.

Preferably the carrier is a parenteral carrier, more preferably a formulation that is isotonic with the blood of the recipient. The conjugate is prepared for storage or administration by mixing it, when it has the desired degree of purity, with physiologically acceptable carriers, excipients, or stabilizers. Such materials are non-toxic to recipients at the dosages and concentrations employed, and will depend on the water solubility of the conjugate. If the conjugate is water soluble, it may be formulated in a buffer such as phosphate or other organic acid salt preferably at a pH of about 7 to 8. If the conjugate is not very soluble in water, it may be prepared as a microemulsion by formulating it with a nonionic surfactant such as Tween, Pluronics, or PEG, e.g., Tween 80, in an amount of 0.04-0.05% (w/v), to increase its solubility.

Optionally other ingredients may be added such as antioxidants, e.g., ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; and sugar alcohols such as mannitol or sorbitol.

The conjugate to be used for therapeutic administration must be sterile. Sterility is readily accomplished by filtration through sterile filtration membranes (e.g., 0.2 micron membranes). The conjugate ordinarily will be stored in lyophilized form or as an aqueous solution or emulsion.

Any reference to the conjugates herein also includes the pharmaceutically acceptable salts of such compounds, and it will be understood that use of certain of the foregoing excipients, carriers, or stabilizers will result in the formation of such salts. Examples of pharmaceutically acceptable salts include those of alkaline earths (e.g., sodium or magnesium), ammonium or NX₄⁺ (wherein X is C₁₋₄ alkyl). Other pharmaceutically acceptable salts include organic carboxylic acids such as acetic, lactic, tartaric, malic, isethionic, lactobionic, and succinic acids; organic sulfonic acids such as methanesulfonic, ethanesulfonic, benzenesulfonic, and p-toluenesulfonic acids; and inorganic acids such as hydrochloric, sulfuric, phosphoric, and sulfamic acids. Physiologically acceptable salts of a compound having a hydroxy group include the anion of said compound in combination with a suitable cation such as Na⁺, NH₄⁺, and NX₄⁺ (wherein X is a C₁₋₄ alkyl group).

Therapeutic conjugate compositions generally are placed into a container having a sterile access port, for example, an intravenous solution bag or vial having a stopper pierceable by a hypodermic injection needle.

The conjugates herein are also suitably administered by sustained release systems. Suitable examples of sustained release compositions include semi-permeable polymer matrices in the form of shaped articles, e.g., films, or microcapsules. Sustained release matrices include polylactides (U.S. Pat. No. 3,773,919, EP 58,481), copolymers of L-glutamic acid and gamma-ethyl-L-glutamate (U. Sidman et al., Biopolymers, 22, 547-556 (1983)), poly(2-hydroxyethyl methacrylate) (R. Langer et al., J. Biomed. Mater. Res., 15: 167-277 (1981), and R. Langer, Chem. Tech., 12: 98-105 (1982)), ethylene vinyl acetate (R. Langer et al., Id.) or poly-D-(-)-3-hydroxybutyric acid (EP 133,988).

Sustained release conjugate formulations also include liposomally entrapped conjugates. Such systems have the advantage that biologically active material can be introduced into tissues by phagocytosis, especially into tissues of the reticuloendothelial system. Liposomes containing conjugates are prepared by methods known per se, including: DE 3,218,121; Epstein et al., Proc. Natl. Acad. Sci. U.S.A., 82: 3688-3692 (1985); Hwang et al., Proc. Natl. Acad. Sci. U.S.A., 77: 4030-4034 (1980); G. Gregoriadis, Liposome Technology, Vol. II. Incorporation of Drugs, Proteins, and Genetic Material, CRC Press, 1984; EP 52,322; EP 36,676; EP 88,046; EP 143,949; EP 142,641; Japanese Pat. Appln. 83-118008; U.S. Pat. Nos. 4,485,045; 4,774,085; and 4,544,545; and EP 102,324. In addition, the liposome may be antibody-coated for increase in uptake by the cells, as taught by Wang and Huang, Proc. Natl. Acad. Sci. U.S.A., 84: 7851-7855 (1987). The liposomes obtained can be stored in the aqueous phase up to several weeks or months after addition of stabilizers, for example, lactose.

The size of the liposomes formed depends, for example, on the structure of the active ingredient and the lipid component, the mixing ratio of the lipid components, and the concentration of these components in the aqueous dispersion. Thus, for example, by increasing or reducing the concentration of the lipid component it is possible to produce aqueous phases having a high content of small or large liposomes. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the liposome-containing aqueous dispersion is suitably mixed with customary thickeners, for example, hydroxypropylcellulose, suitable preservatives, and antioxidants, and can be used in the form of a lotion or gel for application to the skin or mucous membranes. For parenteral use, the aqueous dispersion of the enriched liposomes can be suspended in a suitable carrier liquid, for example, sterile, calcium free, isotonic sodium chloride or glucose solution, optionally buffered to pH 7.2-7.4.

It is estimated that the dose to be applied to a human of about 70 kg weight will range from about 200 mg to 1 g of liposomes containing about 0.1 to 500 mg of entrapped conjugate, respectively. However, the highest and lowest dose of the encapsulated material, the concentration of the phospholipids in the aqueous phase, as well as the proportions of the encapsulating phospholipids, can be varied according to results to be established experimentally in clinical trials, the "effective amount" being thereby tied to the therapeutic results so obtained.

The liposomal pharmaceutical administration system making use of the present invention may consist of a kit of parts set comprising vials or bottles containing the phospholipids and conjugates.

If the antisense oligomer is to be used for cancer treatment, the conjugate therapy may be useful in conjunction with conventional chemotherapeutic agents, such as 5-fluorouracil. If the antisense oligomer is to be used for AIDS treatment, the conjugate therapy may be useful in conjunction with AZT, CD-4, and other experimental AIDS treatment agents.

If the conjugate herein is to be used for diagnostic purposes, it is labeled with a suitable label moiety, typically the oligonucleotide being labeled. Suitable labels include radioactive labels such as ³²P, ¹²⁵I, ³⁵S, or the like, and non-radioactive labels such as, for example, biotin, thyroxine, enzymes such as hydrolases or peroxidases or phosphatases, and various chemiluminescers such as luciferin, or fluorescent compounds such as fluorescein and its derivatives.

One diagnostic technique in which labeled conjugates are useful is in assays for nucleic acid having a predetermined nucleotide sequence in a sample relying on immobilization of the oligomeric probe on a solid support. Immobilizing the probe on a hydrophobic surface through the lipid moiety allows the oligonucleotide to be more accessible to hybridization than it is using, e.g., Southern blots or dot blots. Also, in the instant method, the filter need only be washed with a solution of the conjugate rather than heated to immobilize the nucleotides, as required for Southern blots.

The method herein involves immobilizing the conjugate, in labeled form, on a support, preferably a solid matrix such as an agarose gel or a filter membrane, and contacting the sample with the immobilized conjugate such that the nucleic acid will hybridize with the oligonucleotide portion of the conjugate if the nucleic acid is present in the sample. Conditions for hybridization are well known and include those described in Maniatis, Molecular Cloning: A Laboratory Manual (New York: Cold Spring Harbor Laboratory, 1982). Typically after such hybridization the support is washed to remove non-hybridizing materials and then the presence of labeled oligomers is determined.

In this context, the word "sample" refers to any liquid or biological sample that contains or potentially contains the nucleic acid to be assayed. Thus, this term includes fluids such as human or animal body fluids, e.g., blood, serum, urine, amniotic fluid, tissue extracts, cerebrospinal fluid, and the like.

The above diagnostic method is useful for detection of specific nucleic acid sequences associated with infectious diseases, genetic disorders, or cellular disorders such as cancer, e.g., oncogenes. Genetic diseases include specific deletions and/or mutations in genomic DNA from any organism, such as, e.g., sickle cell anemia, cystic fibrosis, α-thalassemia, β-thalassemia, and the like. Infectious diseases can be diagnosed by the presence in clinic samples of specific DNA sequences characteristic of the causative microorganism. These include bacteria, such as Salmonella, Chlamydia, and Neisseria; viruses, such as the hepatitis viruses; and parasites, such as the Plasmodium responsible for malaria.

If a disease is characterized by the presence or absence of at least one specific restriction site in a specific nucleic acid sequence, such as sickle cell anemia and β-thalassemia, it may be detected by the use of restriction enzymes. Thus, in the above method, after the support is washed, the immobilized conjugate is treated with a restriction endonuclease so as to cleave a restriction site within the oligonucleotide portion of the conjugate, producing labeled and unlabeled oligomer fragments, as described in U.S. Pat. No. 4,725,537 issued February 16, 1988. The labeled oligomer fragments are then detected. Thus, DdeI [Geever et al., Proc. Natl. Acad. Sci., 78: 5081-5085 (1981)] or MstII [Orkin et al., N. Engl. J. Med., 307: 32-36 (1982)] may be employed if the disease is sickle cell anemia.

In another embodiment of diagnosis, a liposome incorporating the conjugate, prepared as described above, and a label (e.g., by encapsulation) is used in the detection of lupus erythematosus in humans. The method involves contacting serum from the human patient with the liposome (where the conjugate acts as the lupus-specific antigen) under conditions such that if lupus autoantibodies are present (as are contained in patients with active lupus), they will bind to the oligonucleotide on the liposome, thereby either stabilizing or destabilizing the liposome; and measuring for the presence or absence of label. If the binding to the oligonucleotide destabilizes the liposome, the label will be released and the amount of label released is measured. If the binding stabilizes the liposome, the label is absent and will not be detected. The assay generally involves one-minute incubation of the serum at room temperature and can be performed with standard microtiter plates, allowing the screening of large numbers of sera, in a short time. The conditions employed for incubation are suitably those used in the colorimetric test for lupus described by Janoff *et al*., Clin. Chem., 29: 1587-1592 (1983) and EP 90,735 published Oct. 5, 1983. These references describe the liposome stabilization embodiment.

For the detection of lupus, preferably the oligonucleotide of the conjugate is double-stranded because it binds better to the antibodies. This may be accomplished by synthesizing a hairpin double-stranded nucleic acid, as is well known to those skilled in the art.

Another manner in which to use the conjugates herein is in the separation, isolation, or purification of a nucleic acid (e.g., oligonucleotides or polynucleotides) from a mixture of components. This procedure takes advantage of the altered physical properties of the oligonucleotide on the conjugate because of the presence of the lipid. The conjugate employed is one that has a nucleotide sequence on the oligonucleotide that is substantially complementary to a sequence contained within the nucleic acid being purified.

In one such use, the lipid of the conjugate is used to immobilize the oligonucleotide on a solid support containing a hydrophobic surface such as plastic, silanized glass, alkyl sepharose or other hydrophobic interaction chromatography support, or an RP-HPLC matrix (e.g., alkyl silica gels such as C8 Vydec). For this purpose, one need only dissolve the conjugate in a buffer such as a phosphate buffer and wash the resulting solution through the column. The lipid will adhere to the column under such aqueous conditions. Then the solid support is treated with the sample containing the complementary target nucleic acid, which hybridizes to the support under conditions that allow such hybridization as are well known to those skilled in the art. After hybridization the support is washed under different temperature or salt conditions than were employed to apply the mixture so as to denature the desired nucleic acid and isolate it. The conjugate can then be recovered from the column by washing it with acetonitrile or other organic solvent.

In another such use of this method to separate nucleic acids the lipid portion of the conjugate is used for transporting the complementary target nucleic acid through a water-non-miscible phase. Thus, the conjugate, having a nucleotide sequence capable of hybridizing to a sequence contained within the oligonucleotide to be separated, is provided in a hydrophobic phase, such as octanol, in a suitable vessel for extraction and transfer from an organic phase to an aqueous phase, such as a U-tube. Likewise, the mixture containing the target nucleic acid is provided in a hydrophilic phase such as water in the vessel. The vessel also contains an aqueous phase without any nucleic acids, preferably pure water, into which the desired nucleic acid is transferred. The conjugate is partially soluble in both the aqueous and hydrophobic phases and its oligonucleotide portion would hybridize to the target strand, under suitable hybridizing conditions, and would then transport that strand through the hydrophobic phase into the pure hydrophilic phase. This procedure represents in effect a specific selective DNA extraction method.

The invention will be more fully understood by reference to the following examples. They should not, however, be construed as limiting the scope of the invention.

### EXAMPLE I

### Preparation and Purification of Conjugate

### A. Preparation of Lipid Salt

1,2-di-O-hexadecyl-rac-glycerol (Sigma) in an amount of 342 mg (0.63 mmol) was evaporated from pyridine twice and then dissolved in 6 ml of pyridine and methylene chloride (1:1). After the flask was filled with argon, the solution, while being stirred at room temperature, was treated with van Boom's reagent (1.25 M solution in dioxane, 0.52 ml) (Marugg et al., Tetrahedron Lett., 27: 2661 (1986)). When the reaction was complete (as determined by thin layer chromatography using EM silica gel 60 F₂₅₄ pre-coated plates visualized with an acidic molybdenum stain where the starting material has an R_{f} of 0.53 and the product has an R_{f} of 0.24), the solution was diluted with methylene chloride (10 ml) and then shaken with several portions of aqueous triethylammonium bicarbonate (TEAB; 1 M). The organic layer was dried over anhydrous sodium sulfate, filtered, and evaporated in vacuo. Column chromatography performed with EM silica gel-60 (70-230 mesh) using SiO₂, 5-20% methanol/methylene chloride with 1% added triethylamine followed by 1% acetic acid in place of triethylamine) followed by additional extractions of the purest fractions with 1 M TEAB afforded 1,2-di-O-hexadecyl-rac-glycero-3-H phosphonate as an off-white triethylammonium salt (76% yield).

¹H NMR spectra were obtained using a Varian VXR-300S spectrometer and were recorded as ppm (d) using TMS as an internal standard. The results are ¹H NMR (d): 6.86 (d, J = 616 Hz, H-P); 3.90 (dd, J = 4.2 and 7.7 Hz, H₂C-0P); 3.60-3.40 (m, 7 H, H₂C-O-HC-H₂C-O-H₂C,); 2.82 (qua, J = 7.2 Hz, 6H, (H₂C)₃-N); 1.54 (m, 4 H, 2(C-H₂C-C-₀); 1.25 (bs, 26 H); 1.19 (t, J = 7.2 Hz, 9 H, (H₃C-C)₃-N); 0.87 (bt, J = 6.3 Hz, 6H, 2(H₃C-C₁₅).

### B. Preparation of Conjugate

The synthesis of polymer-bound nucleotide H-phosphonates was performed on a Biosearch Model 4000 DNA synthesizer using a derivatized controlled-pore glass as the support in the method described in Froehler, B.C. and Matteucci, M.D., Tetrahedron Lett., 27: 469 (1986); Froehler, B.C. *et al*., Nucl. Acids Res., 14: 5399 (1986); Froehler, B.C. and Matteucci, M.D., & Nucleotides, 6: 287 (1987); Garegg, P.J. *et al*., Tetrahedron Lett., 27: 4051 (1986). Detritylation of the oligodeoxynucleotide afforded a free 5' hydroxyl group suitable for conjugation with the lipid hydrogen phosphonate product of section A. This product was dissolved (25 mg/ml) in pyridine/acetonitrile (1:1) and conjugated with the polymer-bound oligodeoxynucleotide using pivaloyl chloride as the activating agent under the coupling conditions described in the above references. Doubling the final coupling time and/or repetition of the coupling cycle had little apparent effect on the yield or purity of the products. The compounds prepared were: EL-T₁₀; EL-AGCTAGCT; EL-AGCTAGCTTTTTAGCTAGCT; EL-CAGTGATGTGT; EL-ACACATCACTG, in which EL represents 1,2-di-O-hexadecyl-3-glycerylphosphate bound to the 5' hydroxyl group of the DNA.

After cleavage and deprotection using the methods in the above references, the products were analyzed using thin-layer chromatography, polyacrylamide gel electrophoresis, RP-HPLC, and enzymatic degradation.

### C. Polyacrylamide gel electrophoresis

EL-DNA samples were dissolved in formamide-containing buffer, heated to 90°C for one minute, and loaded on 15% acrylamide, 7 M urea gels. After running at 50 mA (approximately 400 V) for 2.5 hours, gels were photographed under uv illumination. The lipid-containing EL-DNAs (visualized by UV shadowing) were less mobile than the corresponding lipid-free DNAs and appeared as broad streaks surrounding a major band. In each case, this major band was approximately as mobile as a normal oligomer twice as long as the DNA of the EL-DNA.

Polyacrylamide gel electrophoresis proved to be of little value, as the lipid-containing products appeared as indistinct smears of lower mobility than the corresponding non-lipid DNAs. Comparison of the relative amount of streaking EL-DNA to the normal pattern of DNA bands provided only a very rough measure of lipid incorporation.

### D. Thin-layer chromatography

The samples were run on analytical TLC plates (SiO₂, nPrOH:conc. aq. NH₄OH:H₂O 55:10:35) and visualized both by UV and staining (alcoholic p-anisaldehyde followed by heating).

Using the propanol/ammonia/water eluant, the different species were obtained as distinct, individual spots. The crude synthetic products typically showed two or three spots corresponding to EL-DNA, DNA, and sometimes a trace of unidentified material at lower Rf. The major material (UV and stain) in all cases was the high Rf (0.55-0.70) EL-DNA. The corresponding normal oligomers ran just above or just below the EL-DNAs. The relative locations of the compounds were constant, but their Rf values were variable. In addition to the Rf differences, the color of the spots (produced by reaction with the molybdenum stain) differed. EL-DNA appeared purple, while DNA was dark blue (as was the unidentified minor spot).

### E. Reverse-phase HPLC

RP-HPLC was performed using a Waters Model 510 system and a 10 um C8 Partisil-10, 4.6 x 250 mm analytical column. RP-HPLC samples were loaded in 100% "A" buffer (25 mM aqueous triethylammonium phosphate (TEAP), pH 7.0, 5% CH₃CN). Increasing amounts of "B" buffer (25 mM aqueous TEAP, pH 7.0, 75% CH₃CN) were used to elute the samples from the column at a flow rate of 2.0 ml/min. Peaks were detected at 254 nm and were collected using an automated fraction collector.

Elution with the aqueous TEAP buffer with an acetonitrile gradient afforded good separation of EL-DNA from the small amount of contaminating normal DNA present in each reaction mixture. Under these conditions, DNA lacking the lipid eluted at 10-20% "B" depending on their length. In contrast to normal DNA, longer lipooligonucleotides were eluted before the shorter ones. Following are elution data for the five EL-DNAs (% "B"): EL-AGCTAGCTTTTTAGCTAGCT (57%); EL-CAGTGATGTGT (62%); EL-ACACATCACTG (64%); EL-T₁₀ (65%); EL-AGCTAGCT (68%).

This technique gave the most detailed product information. Using TEAP buffer with an acetonitrile gradient, the presence or absence of lipid in the synthetic material was immediately obvious. In every case, there was a small amount of DNA (and the usual distribution of shorter oligomers) eluting at low acetonitrile percentages and a greater amount of late-eluting EL-DNA. A small analytical HPLC column generally produced a single, broad, tailing peak of lipid-DNA material. After the HPLC materials were isolated, further thin-layer chromatography experiments were run confirming that the late-eluting HPLC peaks and the high Rf, purple-staining TLC spots were identical.

### F. Enzymatic Digestion

Further characterization of one EL-DNA (EL-CAGTGATGTGT) was carried out by enzymatic digestion. Snake venom phosphodiesterase (Boehringer Mannheim GmbH) catalyzed nearly complete hydrolysis of the DNA backbone (36°C, 30 min., 50 mM Tris, pH 8, 10 mM MgCl₂). Bovine spleen phosphodiesterase (Sigma), however, left the lipid-DNA intact (37°C, 1.5 hours, 50 mM Tris, pH 8, 10 mM MgCl₂). These results are as expected for a DNA that bears a free hydroxyl group at the 3' terminus but is blocked at the 5' end. EL-CAGTGATGTGT was found to be incompetent as a substrate for both phospholipase C (Sigma, from Bacillus cereus) and phospholipase D (Sigma, from cabbage) under standard conditions (100 mM Tris, pH 8, 10 mM CaCl₂, 36°C). RP-HPLC analysis of the digestion products showed only intact EL-DNA with each of the enzymes.

### G. Thermal Denaturation Profile

Samples containing normal DNA (CAGTGATGTGT/ACACATCACTG), EL-DNA (EL-CAGTGATGTGT/EL-ACACATCACTG), or a mixture of DNA/EL-DNA (EL-CAGTGATGTGT/ACACATCACTG and CAGTGATGTGT/EL-ACACATCACTG) in 1 ml of buffer (100 mM NaCl, 10 mM Na₂HPO₄, 1 mM EDTA, pH 7.2; each strand 2 µM) were placed in a masked 1-cm cuvette. All the samples were desalted (SEP-PAK C-18 minicolumn), HPLC-purified material. The insulated cell compartment was warmed from 10 to 80°C in 1°C increments with equilibration for 1 min. after attaining each temperature. UV absorbance at 260 nm was recorded automatically as a function of temperature.

The Tₘs for EL-CAGTGATGTGT/ACACATCACTG (38°C) and CAGTGATGTGT/EL-ACACATCACTG (33°C) were decreased relative to that of the normal duplex CAGTGATGTGT/ACACATCACTG (41°C). There have been a number of reports of Tₘ decreases for DNA-ligand conjugates. See, for example, Kempe et al., Nucl. Acids Res., 13: 45 (1985). The varying degree of Tₘ depression may simply reflect the differences in GC vs. AT content at the lipid-bearing end of each duplex. More interesting is that with lipid on both DNA strands (EL-CAGTGATGTGT/EL-ACACATCACTG; Tₘ 50°C) the Tₘ is markedly higher than for lipid-free DNA. These results may indicate the formation of some higher-order lipid structures when lipid is present at each end of the duplex that are not accessible to duplexes bearing only a single lipid.

### EXAMPLE II

### Transfection Assay of Conjugate

It is known that NIH/3T3 mouse fibroblasts can be transformed by ras oncogenes using calcium chloride transfection (see Graham and Van der Eb, Virology, 52: 456-467 (1973) and Wigler et al., Proc. Natl. Acad. Sci. U.S.A., 76: 1373-1376 (1979)). The transformed cells form foci of refringent cells that have lost contact inhibition and can grow in athymic mice.

In this prophetic example, the human ras oncogene (isolated from a plasmid, for example) is covalently coupled to a phospholipid by any of the techniques described above, including nitrogen linkage, or preferably is ligated to a lipid-oligonucleotide conjugate. In the latter case, a linker is constructed that is complementary to and spans the 3' end of the oligonucleotide of the conjugate to the 5' end of the human ras oncogene (which is contained in a linearized plasmid). This oligonucleotide portion of the conjugate, the linker, and the linearized plasmid are phosphorylated by standard kinase techniques and ligated together using T4 ligase.

Confluent monolayers of NIH/3T3 cells are washed twice with Dulbecco's Modified Eagle's medium (DMEM) without serum and incubated in this DMEM medium for four hours. Then, one of the lipid-ras conjugates is added to the medium and allowed to incubate for three hours at 37°C. Complete DMEM medium with 10% fetal calf serum is then added to the Petri dish, in an amount of 10:1 (v/v), and allowed to stand overnight at 37°C. The next day the cells are split in 1/20, 1/40, and 1/80 dilutions and left growing for 17 days, after which the foci are scored.

The ras oncogene can be internalized into the cell, brought to the nucleus, and expressed to yield the ras oncogene product, showing that the conjugates can transform NIH/3T3 cells in a stable fashion.

In summary, the present invention is directed to conjugates of oligonucleotides and lipids for transporting, targeting and internalizing the oligonucleotides to and within appropriate cells, generally by endocytosis. After internalization, the conjugate is cleaved, for example, by cellular lipases that recognize an appropriate cleavage site on the conjugate. Thus, the conjugate herein suitably acts as a cytospecific drug capable of specific delivery to a cell of an oligomer expressing a polypeptide having various types of activities, for example, therapeutic, prophylactic, antiviral, cytotoxic, etc. In addition, the oligomer may affect the regulatory mechanism of the cell, complement a genetic defect, or serve as a marker.

## Claims

1. A covalent conjugate of a lipid and an oligonucleotide, or a pharmaceutically acceptable salt thereof, wherein the lipid is covalently linked through phosphate to the 5'- end of the oligonucleotide, and is selected from steroids, glycerides and glyceryl ethers; with the proviso that the conjugate is not nonathymidyluridine modified at the 5'-phosphate with cholesterol.

2. A conjugate or salt according to claim 1 wherein the lipid is a glyceride or glyceryl ether of the formula 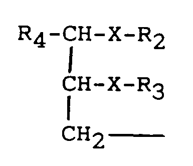 wherein R₂, R₃ and R₄ are independently selected from H, C₁-C₃₀ alkyl, C₂-C₃₀ mono-, di-, or polyunsaturated alkyl, C₃-C₈ cycloalkyl or C₄-C₈ mono-, di-, or polyunsaturated cycloalkyl group and X is O or -O.CO-.

3. A conjugate or salt according to claim 1 or claim 2 wherein the conjugate is adapted to be cleaved by a lipase.

4. A conjugate or salt according to any preceding claim wherein the oligonucleotide moiety is selected from 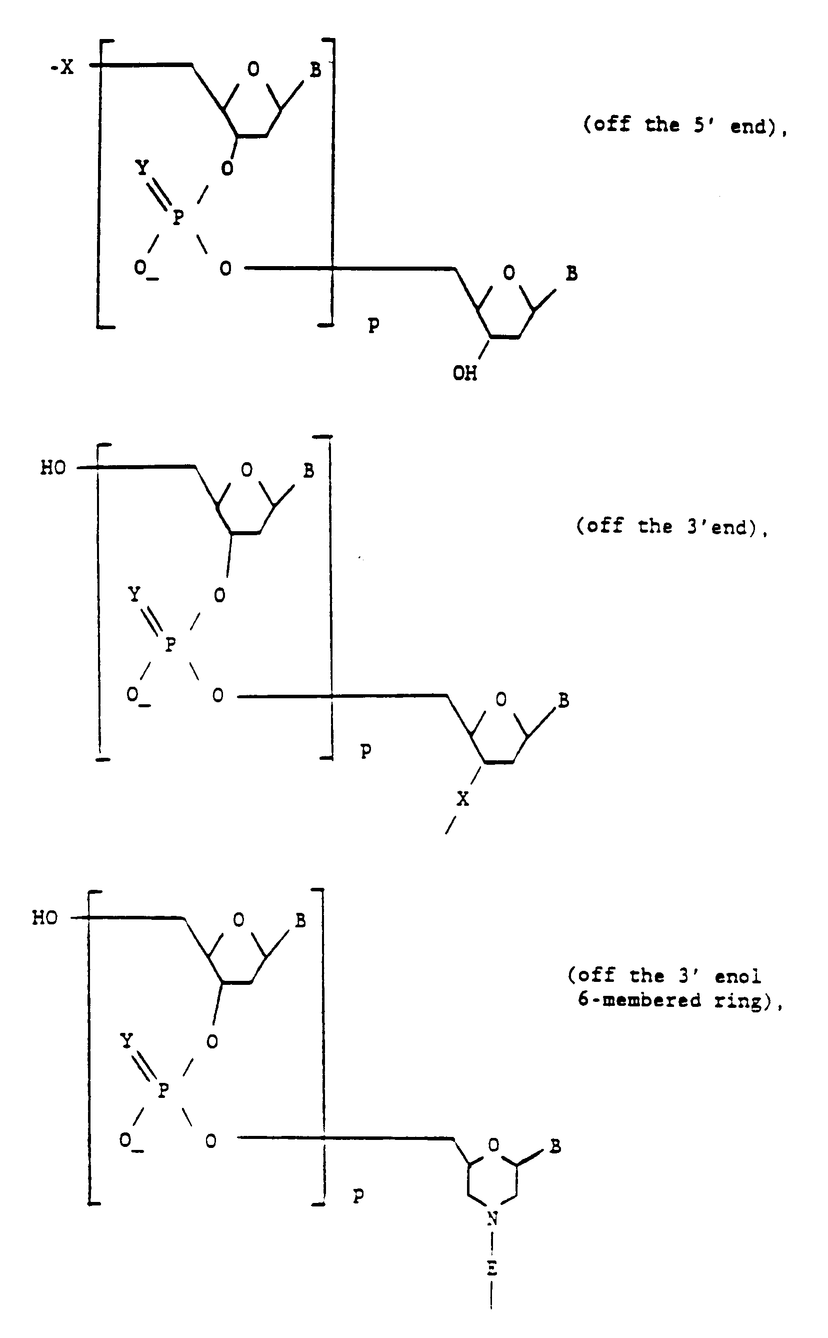 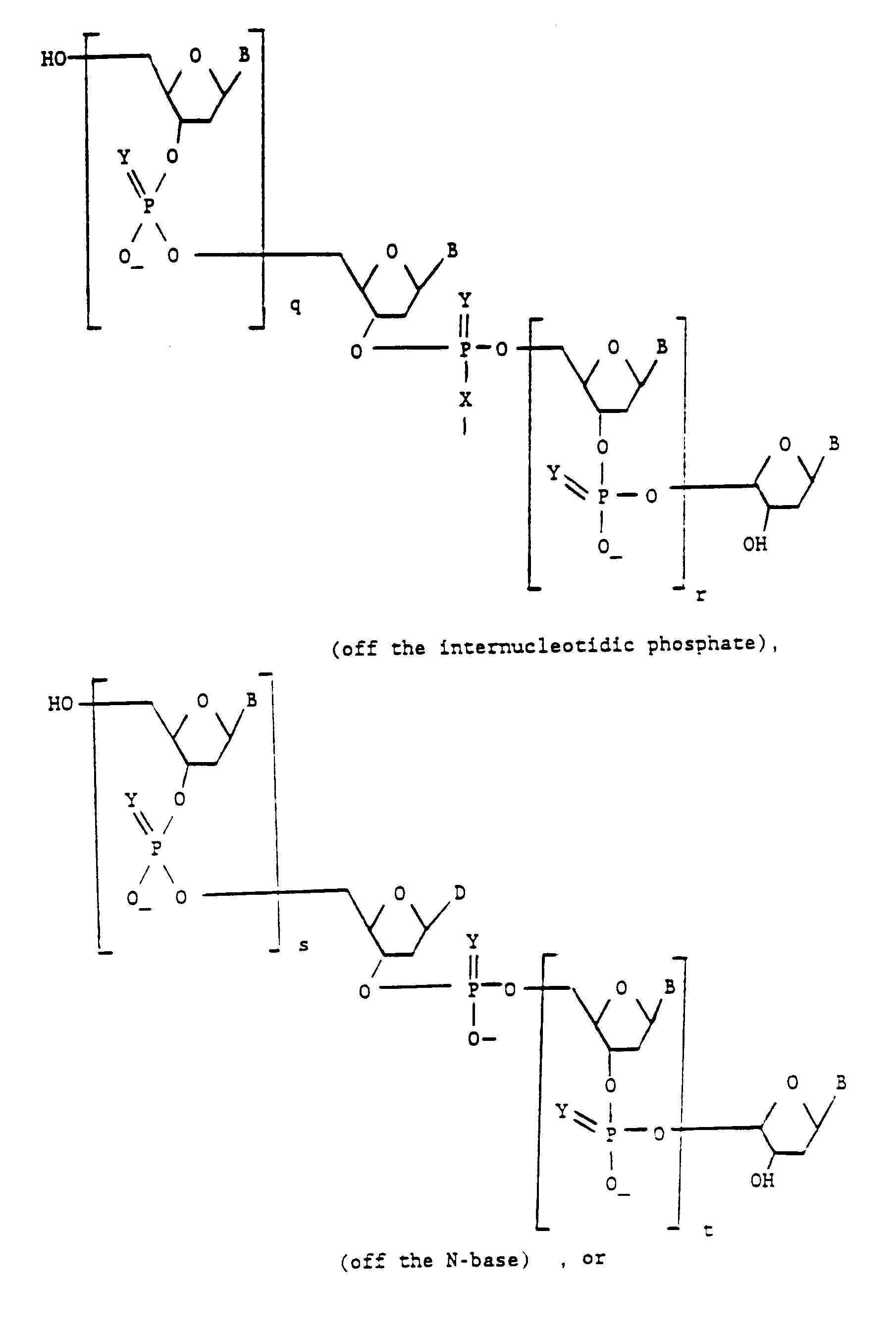 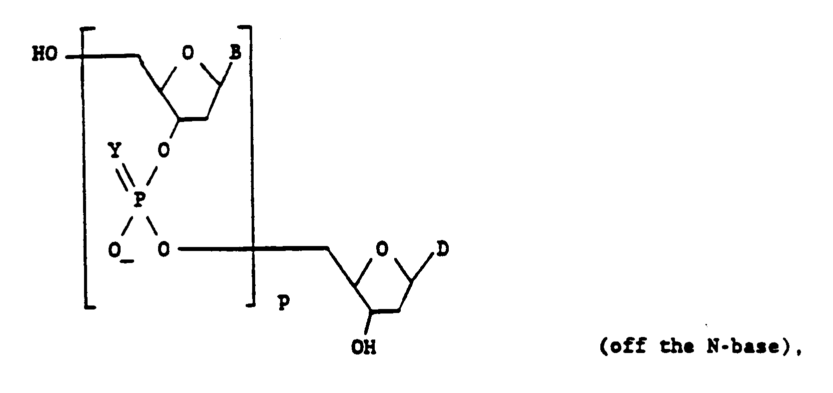 where B is a deprotected base, p is an integer from about 5 to 30, q and r are integers from about 1 to 28, provided that r + q is from about 4 to 29, s and t are integers from 0 to about 29, provided that s + t is from about 4 to 29, E is X or ZX, and D is selected from the group consisting of (where the bond from the ring nitrogens is attached to the sugar moiety of the oligonucleotide):

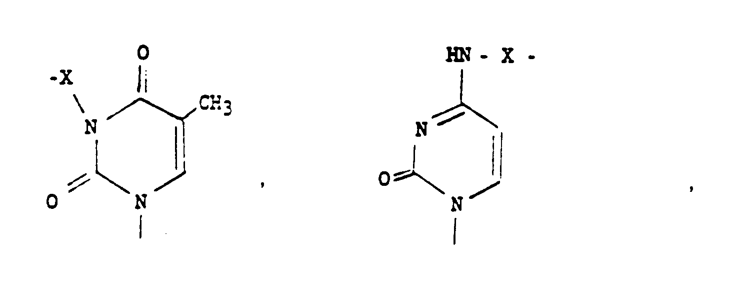

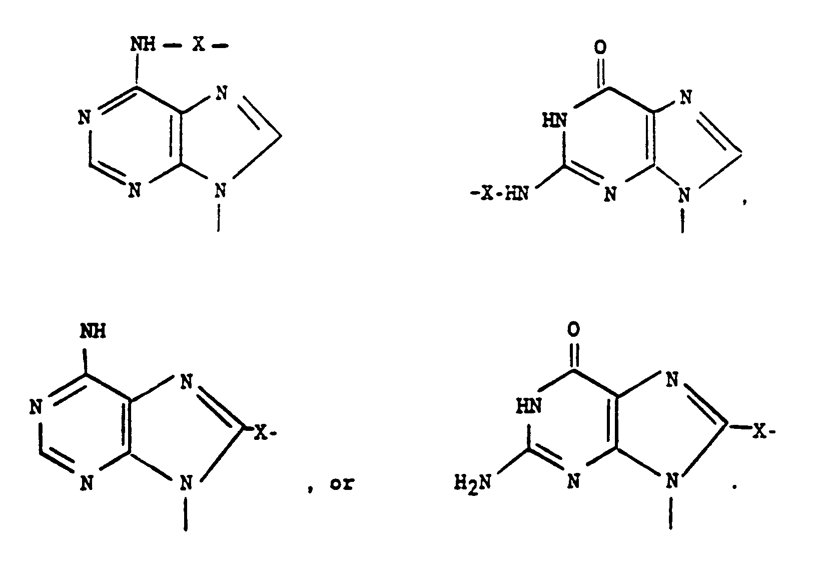

5. A conjugate or salt according to any preceding claim wherein the oligonucleotide moiety is connected through the N6 of an adenine residue or the N4 of a cytosine residue.

6. A conjugate or salt according to any preceding claim wherein at least one base is a substantially planar pyrimidinone base.

7. A conjugate or salt according to any preceding claim wherein the oligonucleotide comprises a nucleotide sequence sufficiently complementary to a pathogenic nucleic acid or an oncogene to hybridize thereto and/or representing, or capable of hybridizing to, a cleavage site specifically recognised by an enzyme endogenous to a host cell to which the conjugate is targeted and/or representing, or capable of hybridizing to, an mRNA splice site.

8. A conjugate or salt according to any preceding claim for use as a medicament.

9. A method for the assay of a nucleic acid having a predetermined nucleotide sequence in a sample comprising:
(a) providing a labelled conjugate according to any of claims 1-7 that has a nucleotide sequence capable of hybridizing to the predetermined sequence;
(b) immobilizing the labeled conjugate on a support;
(c) contacting the sample with the immobilized conjugate under conditions that would cause hybridization of the nucleic acid with the oligonucleotide portion of the conjugate if the nucleic acid is present in the sample; and
(d) detecting the presence of labeled oligomers.

10. The method of claim 9 which further comprises, after step (c), the step of contacting the immobilized conjugate under conditions of digestion with a restriction endonuclease that is capable of cleaving the oligonucleotide portion of the conjugate so as to produce labeled and unlabeled oligomer fragments; and wherein in step (d) the oligomers are oligomer fragments.

11. A method for separating an oligonucleotide from a mixture, which method comprises:
(a) providing the conjugate of any of claims 1-7 having a nucleotide sequence capable of hybridizing to a sequence contained within the oligonucleotide to be separated;
(b) immobilizing the conjugate on a support;
(c) contacting the mixture with the immobilized conjugate, under conditions causing hybridization of the oligonucleotide of the mixture with the oligonucleotide portion of the conjugate; and
(d) separating the hybridized oligonucleotides.

12. A method for separating an oligonucleotide from a mixture, which method comprises:
(a) providing the conjugate according to any of claims 1-7 having a nucleotide sequence capable of hybridizing to a sequence contained within the oligonucleotide to be separated in a hydrophobic phase;
(b) providing the mixture in a hydrophilic phase;
(c) contacting the phase containing the mixture with the phase containing the conjugate, under conditions causing hybridization of the oligonucleotide of the mixture with the oligonucleotide portion of the conjugate; and
(d) extracting from the phase containing the mixture the hybridized oligonucleotides and transporting them to a separate hydrophilic phase.

13. A liposome comprising the conjugate of any of claims 1-7 and a label moiety.

14. A method for detecting lupus erythematosus in a human comprising contacting a serum sample from the human with the liposome of claim 13, said contacting being such that antibodies in the serum bind to the oligonucleotide on the liposome, so as to alter the stability of the liposome; and measuring for the presence or absence of the label.

15. The method of claim 14 wherein the antibodies bind to the oligonucleotide on the liposome so as to release the label and wherein the amount of the label released is measured.

16. Use of a covalent conjugate of a lipid and an oligonucleotide in the manufacture of a pharmaceutical composition for treatment of a disease associated with a pathogenic nucleic acid or oncogene within a patient's cells.

17. A covalent conjugate of a lipid and an oligonucleotide, or a salt thereof, for use as a medicament for treatment of a disease associated with a pathogenic nucleic acid or oncogene within a patient's cells, wherein the oligonucleotide has a nucleotide sequence sufficiently complementary to said pathogenic nucleic acid or oncogene to hybridise thereto.

18. A conjugate or salt according to claim 17 which is according to any of claims 1 to 7.

## Patentansprüche

1. Kovalentes Konjugat aus einem Lipid und einem Oligonukleotid, oder ein pharmazeutisch akzeptables Salz davon, worin das Lipid über Phosphat kovalent mit dem 5'-Ende des Oligonukleotids verbunden und aus Steroiden, Glyceriden und Glyceryläthern ausgewählt ist; mit der Maßgabe, daß das Konjugat nicht am 5'-Phosphat mit Cholesterin modifiziertes Nonathymidyluridin ist.

2. Konjugat oder Salz nach Anspruch 1, worin das Lipid ein Glycerid oder Glyceryläther der Formel 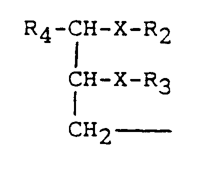 ist, worin R₂, R₃ und R₄ unabhängig voneinander aus H, C₁-C₃₀-Alkyl, mono-, di- oder polyungesättigtem C₂-C₃₀-Alkyl, C₃-C₈-Zykloalkyl oder mono-, di- oder polyungesättigten C₄-C₈-Zykloalkylgruppen ausgewählt sind und X O oder -O.CO- ist.

3. Konjugat oder Salz nach Anspruch 1 oder 2, worin das Konjugat so ausgebildet ist, daß es durch eine Lipase gespalten wird.

4. Konjugat oder Salz nach einem der vorhergehenden Ansprüche, worin die Oligonukleotidgruppe aus 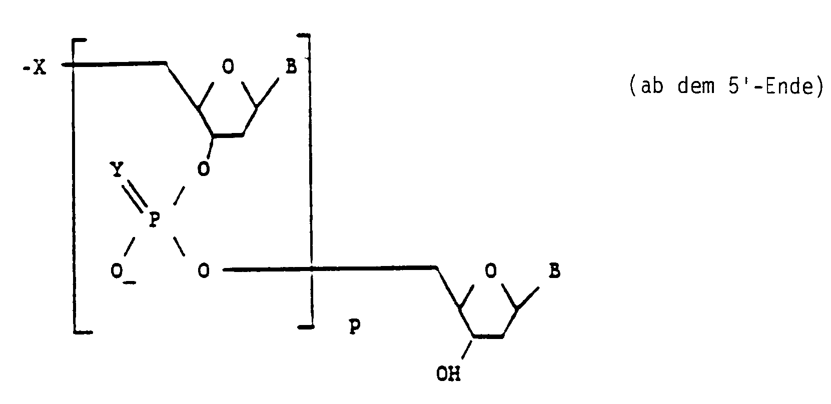 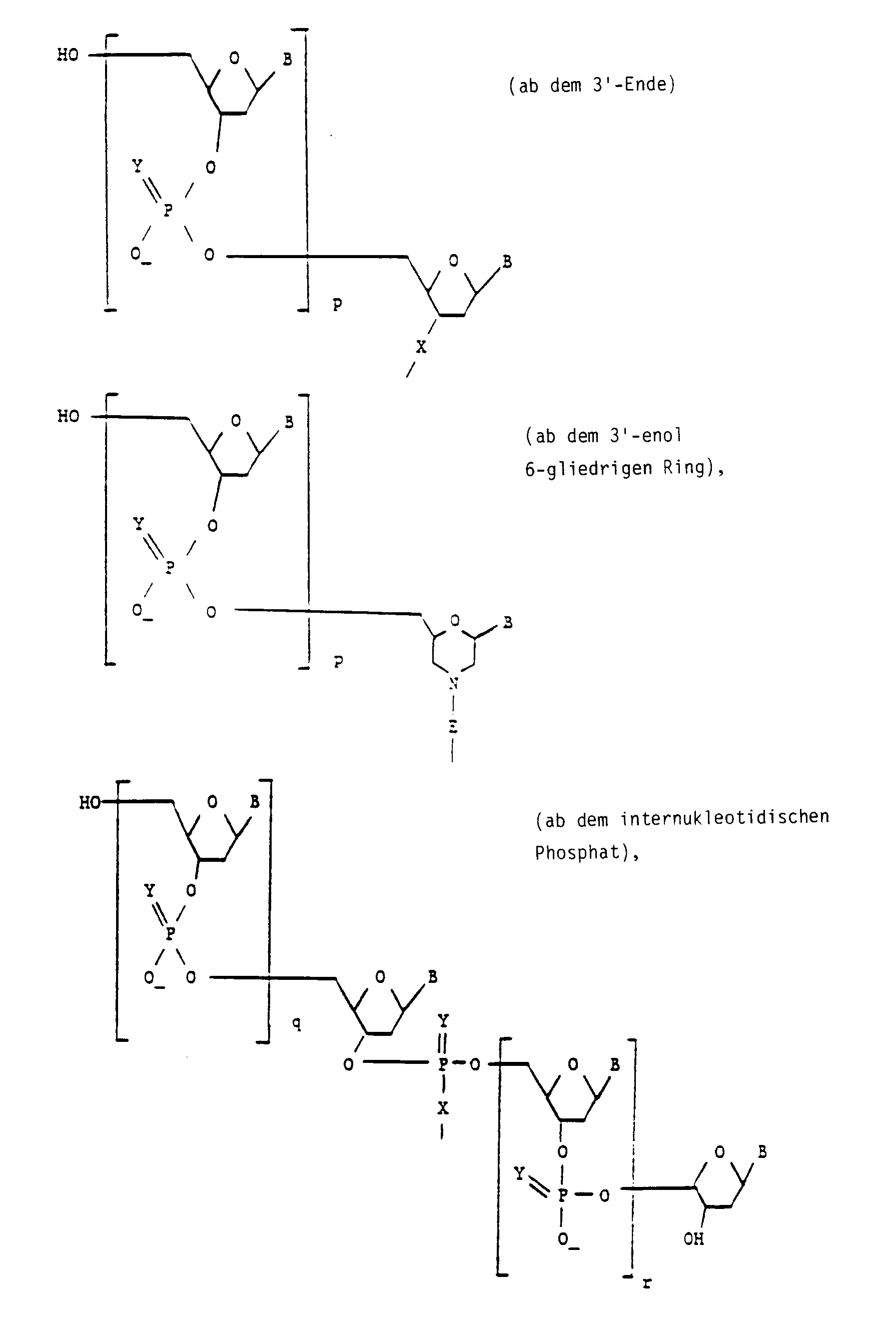 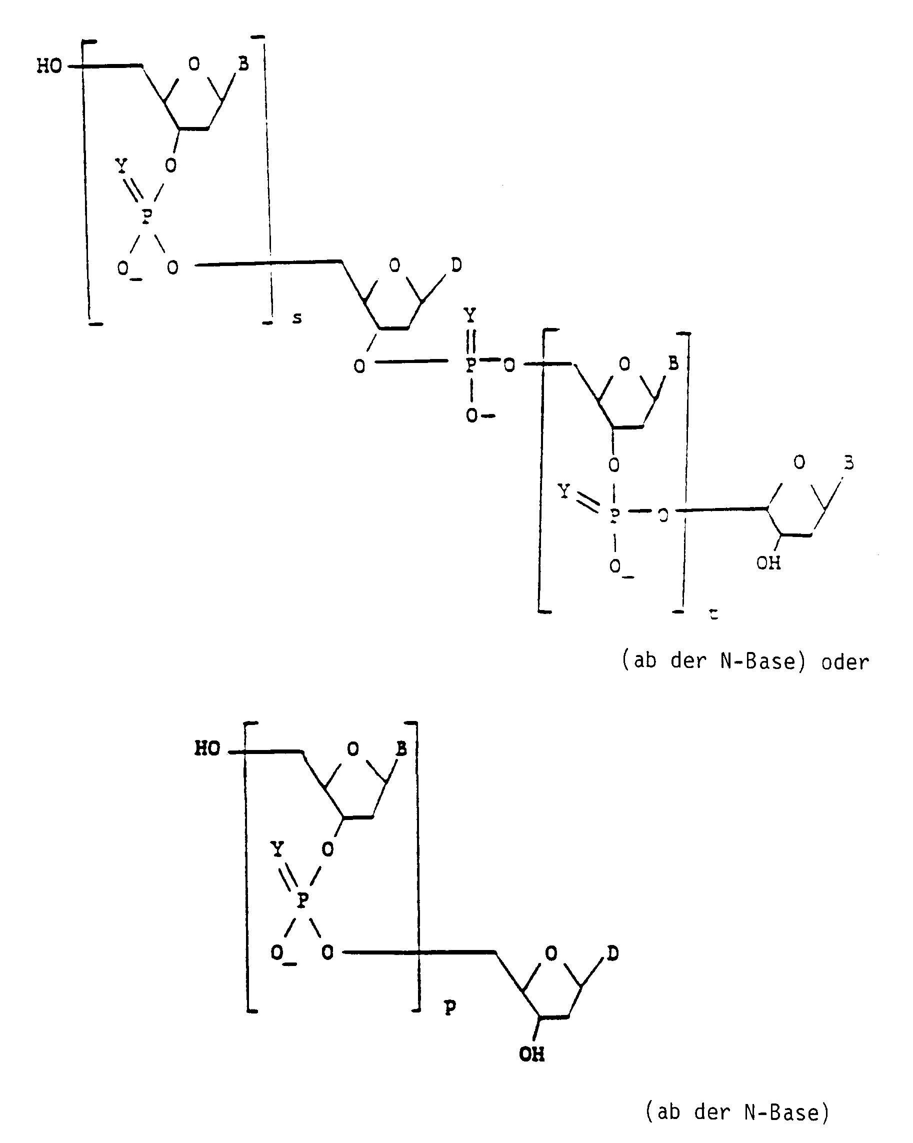 ausgewählt ist, worin B eine Base ist, von der die Schutzgruppe entfernt wurde, p eine ganze Zahl von etwa 5 bis 30 ist, q und r ganze Zahl von etwa 1 bis 28 sind, mit der Maßgabe, daß r + q von etwa 4 bis 29 ausmacht, s und t ganze Zahlen von 0 bis etwa 29 sind, mit der Maßgabe, daß s + t von etwa 4 bis 29 ausmacht, E X oder ZX ist und D aus der Gruppe ausgewählt ist, die besteht aus (wobei die Bindung von den Ringstickstoffen an der Zuckergruppe des Oligonukleotids befestigt ist):

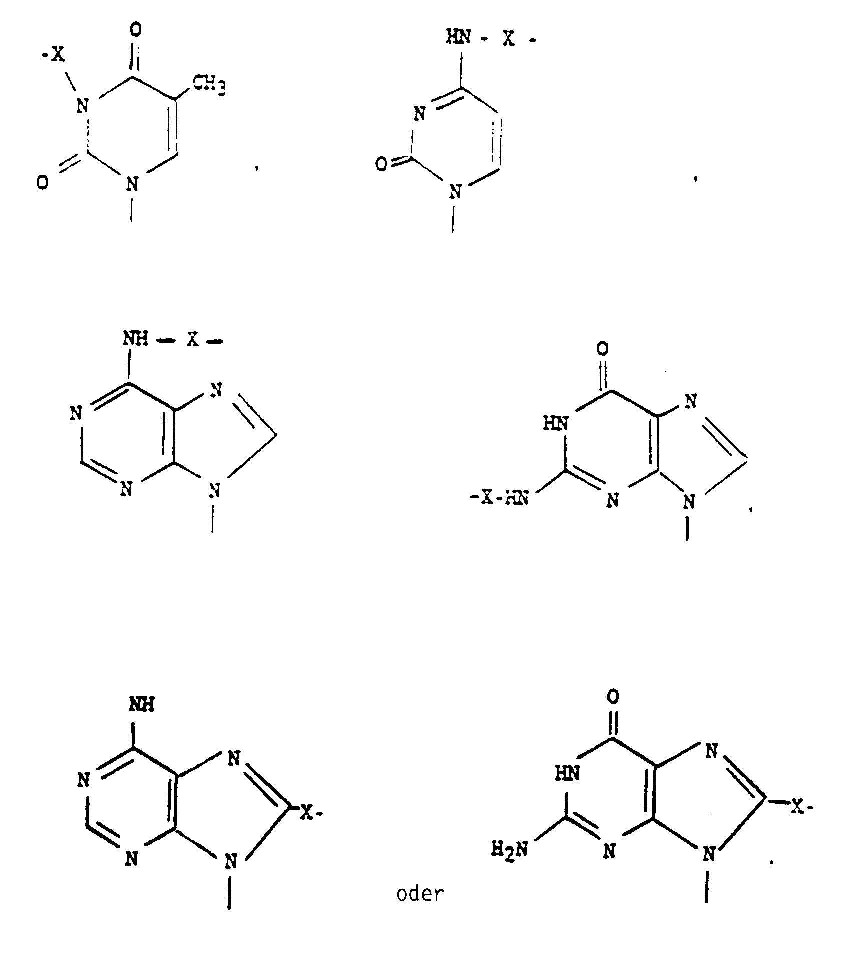

5. Konjugat oder Salz nach einem der vorhergehenden Ansprüche, worin die Oligonukleotidgruppe über den N6 eines Adeninrestes oder den N4 eines Zytosinrestes verbunden ist.

6. Konjugat oder Salz nach einem der vorhergehenden Ansprüche, worin zumindest eine Base eine im wesentlichen planare Pyrimidinonbase ist.

7. Konjugat oder Salz nach einem der vorhergehenden Ansprüche, worin das Oligonukleotid eine Nukleotidsequenz enthält, die zu einer pathogenen Nukleinsäure oder einem Onkogen ausreichend komplementär ist, um damit zu hybridisieren und/oder das eine Spaltungsstelle darstellt oder zur Hybridisierung damit fähig ist, die von einem Enzym spezifisch erkannt wird, das für eine Wirtszelle endogen ist, auf die das Konjugat abzielt, und/oder eine mRNA-Spleißstelle darstellt oder zur Hybridisierung damit fähig ist.

8. Konjugat oder Salz nach einem der vorhergehenden Ansprüche zur Verwendung als ein Medikament.

9. Verfahren für ein Assay einer Nukleinsäure mit einer vorbestimmten Nukleotidsequenz in einer Probe, umfassend:
(a) das Schaffen eines markierten Konjugats nach einem der Ansprüche 1 bis 7, das eine Nukleotidsequenz aufweist, die zum Hybridisieren mit der vorbestimmten Sequenz fähig ist;
(b) das Immobilisieren des markierten Konjugats auf einem Träger;
(c) das In-Kontakt-Bringen der Probe mit dem immobilisierten Konjugat unter Bedingungen, die Hybridisierung der Nukleinsäure mit dem Oligonukleotidabschnitt des Konjugats verursachen würden, wenn die Nukleinsäure in der Probe vorhanden ist; und
(d) das Nachweisen der Gegenwart von markierten Oligomeren.

10. Verfahren nach Anspruch 9, das nach Schritt (c) weiters einen Schritt umfaßt, bei dem das immobilisierte Konjugat unter Bedingungen der Digestion mit einer Restriktionsendonuklease in Kontakt gebracht wird, die fähig ist, den Oligonukleotidabschnitt des Konjugats zu spalten, um markierte und unmarkierte Oligomerfragmente zu erzeugen; und worin die Oligomeren in Schritt (d) Oligomerfragmente sind.

11. Verfahren zum Abtrennen eines Oligonukleotids aus einer Mischung, welches Verfahren umfaßt:
(a) das Schaffen des Konjugats nach einem der Ansprüche 1 bis 7 mit einer Nukleotidsequenz, die zur Hybridisierung mit einer innerhalb des abzutrennenden Oligonukleotids enthaltenen Sequenz fähig ist;
(b) das Immobilisieren des Konjugats auf einem Träger;
(c) das In-Kontakt-Bringen der Mischung mit dem immobilisierten Konjugat unter Bedingungen, die das Hybridisieren des Oligonukleotids der Mischung mit dem Oligonukleotidabschnitt des Konjugats verursachen; und
(d) das Abtrennen der hybridisierten Oligonukleotide.

12. Verfahren zum Abtrennen eines Oligonukleotids aus einer Mischung, welches Verfahren umfaßt:
(a) das Schaffen des Konjugats nach einem der Ansprüche 1 bis 7 mit einer Nukleotidsequenz, die zum Hybridisieren mit einer im abzutrennenden Oligonukleotid enthaltenen Sequenz fähig ist, in einer hydrophoben Phase;
(b) das Schaffen der Mischung in einer hydrophilen Phase;
(c) das In-Kontakt-Bringen der die Mischung enthaltenden Phase mit der das Konjugat enthaltenden Phase unter Bedingungen, welche die Hybridisierung des Oligonukleotids der Mischung mit dem Oligonukleotidabschnitt des Konjugats bewirken; und
(d) das Extrahieren der hybridisierten Oligonukleotide aus der die Mischung enthaltenden Phase und ihren Transport zu einer getrennten hydrophilen Phase.

13. Liposom, welches das Konjugat nach einem der Ansprüche 1 bis 7 und eine Markergruppe enthält.

14. Verfahren zum Nachweisen von Lupus erythematosus bei einem Menschen, welches das In-Kontakt-Bringen einer Serumprobe aus dem Menschen mit dem Liposom nach Anspruch 13 umfaßt, wobei das genannte In-Kontakt-Bringen so erfolgt, daß Antikörper im Serum sich an das Oligonukleotid am Liposom binden, um die Stabilität des Liposoms zu verändern; und das Messen in Hinblick auf die Gegenwart oder Abwesenheit des Markers.

15. Verfahren nach Anspruch 14, worin die Antikörper sich an das Oligonukleotid auf dem Liposom binden, um den Marker freizusetzen, und worin die Menge an freigesetztem Marker gemessen wird.

16. Verwendung eines kovalenten Konjugats aus einem Lipid und einem Oligonukleotid bei der Herstellung einer pharmazeutischen Zusammensetzung zur Behandlung einer Krankheit, die mit einer/einem pathogenen Nukleinsäure oder Onkogen innerhalb der Zellen eines Patienten assoziiert ist.

17. Kovalentes Konjugat aus einem Lipid und einem Oligonukleotid, oder ein Salz davon, zur Verwendung als Medikament zur Behandlung einer Erkrankung, die mit einer/einem pathogenen Nukleinsäure oder Onkogen innerhalb der Zellen eines Patienten assoziiert ist, worin das Oligonukleotid eine Nukleotidsequenz aufweist, die mit der/dem genannten pathogenen Nukleinsäure oder Onkogen ausreichend komplementär ist, um damit hybridisieren.

18. Konjugat oder Salz nach Anspruch 17, gemäß einem der Ansprüche 1 bis 7.

## Revendications

1. Conjugué covalent d'un lipide et d'un oligonucléotide, ou un de ses sels pharmaceutiquement acceptables, dans lequel le lipide est lié par covalence par l'intermédiaire d'un phosphate à l'extrémité 5' de l'oligonucléotide, et est choisi entre des stéroïdes, des glycérides et des éthers de glycéryle ; sous réserve que le conjugué ne consiste pas en nonathymidyluridine modifiée au niveau du phosphate en position 5' avec le cholestérol.

2. Conjugué ou sel suivant la revendication 1, dans lequel le lipide est un glycéride ou éther de glycéryle de formule 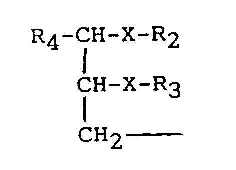 dans laquelle R₂, R₃ et R₄ sont choisis indépendamment entre H, des groupes alkyle en C₁ à C₃₀, alkyle mono-, di- ou polyinsaturés en C₂ à C₃₀, cycloalkyle en C₃ à C₈ ou cycloalkyle mono-, di- ou polyinsaturés en C₄ à C₈ et X représente O ou un groupe -O-CO-.

3. Conjugué ou sel suivant la revendication 1 ou la revendication 2, le conjugué étant apte à un clivage par une lipase.

4. Conjugué ou sel suivant l'une quelconque des revendications précédentes, dans lequel le groupement oligonucléotidique est choisi entre les groupements 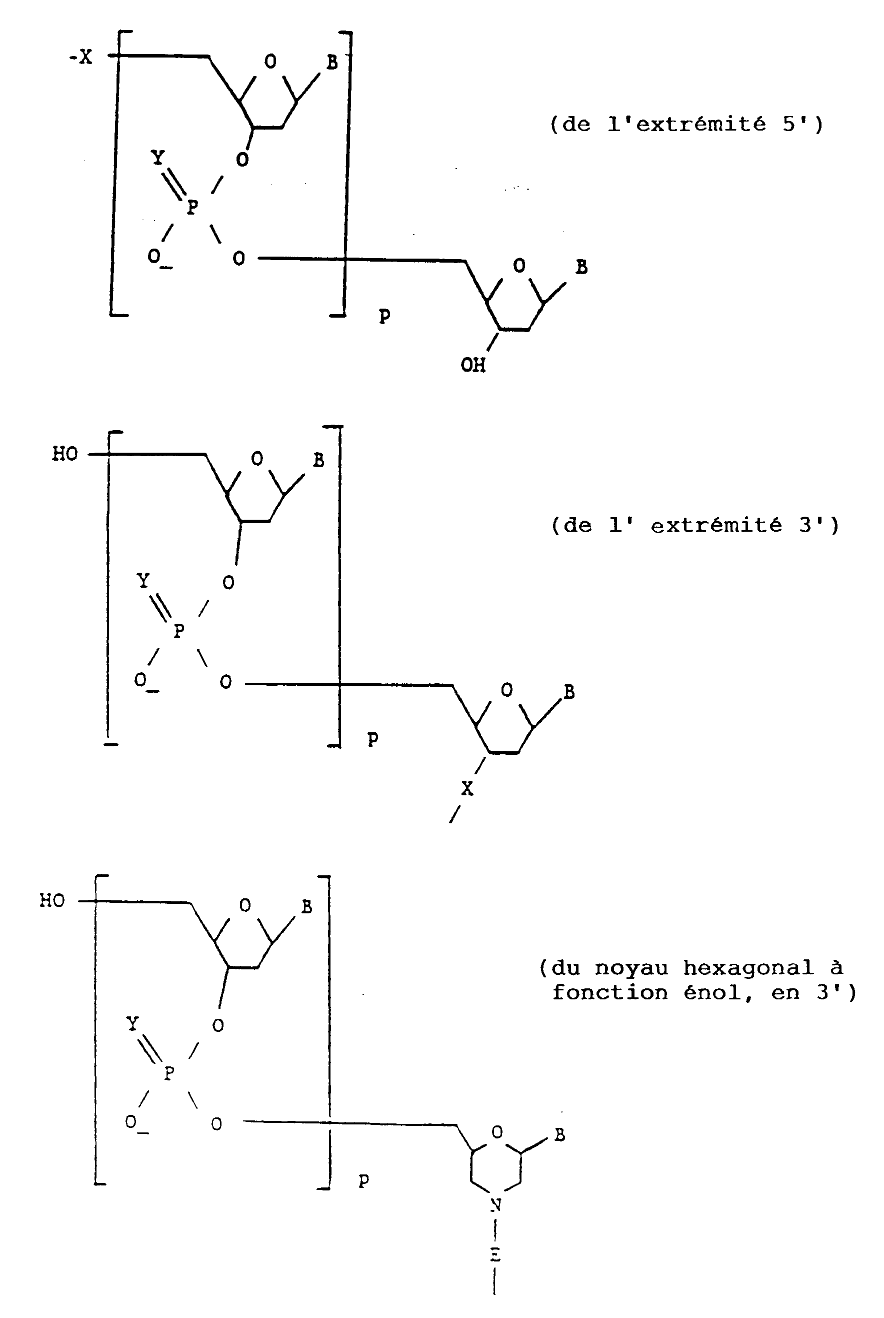 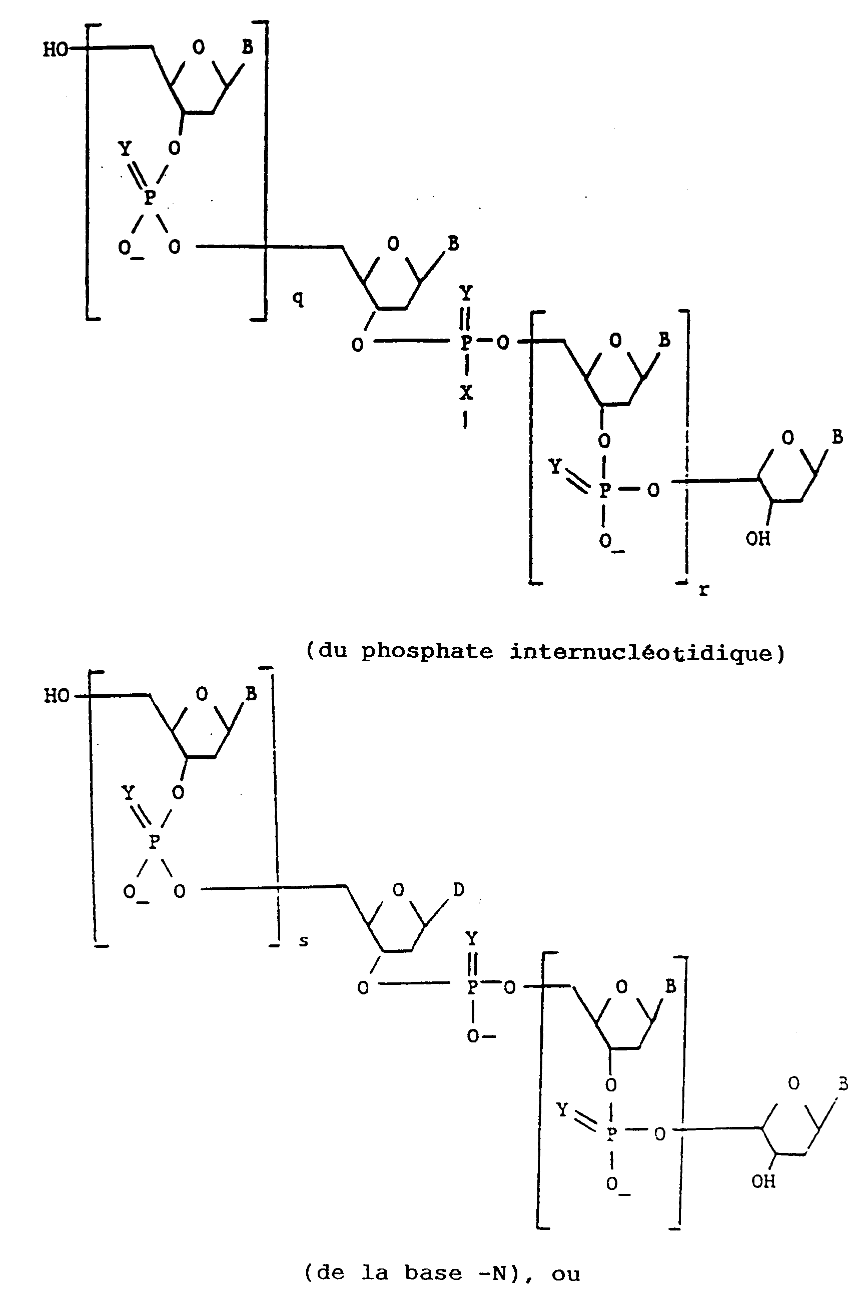 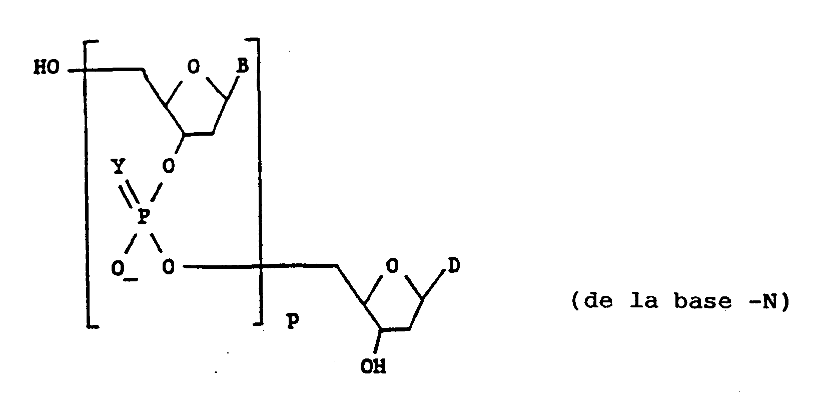 dans lesquels B représente une base non protégée, p est un nombre entier d'environ 5 à 30, q et r sont des nombres entiers d'environ 1 à 28, sous réserve que la somme r + q possède une valeur d'environ 4 à 29, s et t sont des nombres entiers de 0 à environ 29, sous réserve que la somme s + t possède une valeur d'environ 4 à 29, E représente un groupe X ou ZX, et D est choisi entre les groupes suivants (dans lesquels la liaison partant de l'atome d'azote du noyau est fixée au groupement glucidique de l'oligonucléotide :

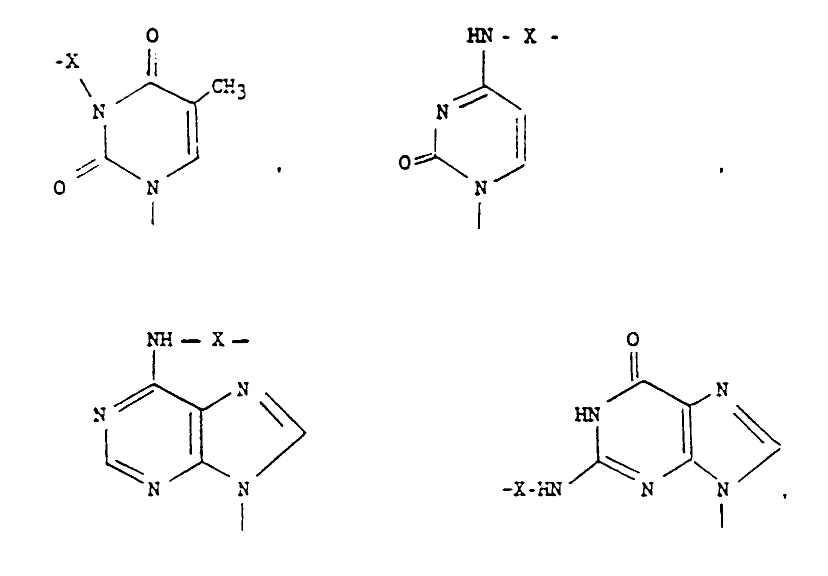

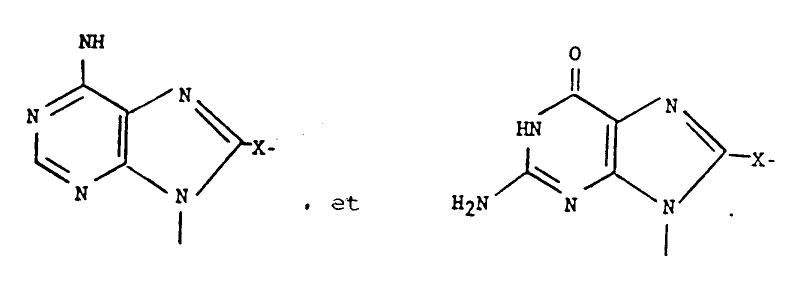

5. Conjugué ou sel suivant l'une quelconque des revendications précédentes, dans lequel le groupement oligonucléotidique est connecté par l'intermédiaire de l'atome N6 d'un résidu adénine ou de l'atome N4 d'un résidu cytosine.

6. Conjugué ou sel suivant l'une quelconque des revendications précédentes, dans lequel au moins une base consiste en une pyrimidinone pratiquement plane.

7. Conjugué ou sel suivant l'une quelconque des revendications précédentes, dans lequel l'oligonucléotide comprend une séquence de nucléotides suffisamment complémentaire d'un acide nucléique pathogène ou d'un oncogène pour s'hybrider à celui-ci, et/ou représentant, ou étant capable de s'hybrider à, un site de clivage reconnu spécifiquement par un enzyme endogène à une cellule-hôte vers laquelle est dirigé le conjugué, et/ou représentant, ou étant capable de s'hybrider à, un site d'épissage d'ARNm.

8. Conjugué ou sel suivant l'une quelconque des revendications précédentes, destiné à être utilisé comme médicament.

9. Procédé d'analyse d'un acide nucléique possédant une séquence de nucléotides prédéterminée dans un échantillon, comprenant les étapes consistant :
(a) à prendre un conjugué marqué suivant l'une quelconque des revendications 1 à 7, qui possède une séquence de nucléotides capable de s'hybrider à la séquence prédéterminée ;
(b) à immobiliser le conjugué marqué sur un support ;
(c) à mettre en contact l'échantillon avec le conjugué immobilisé dans des conditions qui provoqueraient une hybridation de l'acide nucléique avec la portion oligonucléotidique du conjugué dans le cas de la présence de l'acide nucléique dans l'échantillon ; et
(d) à détecter la présence d'oligomères marqués.

10. Procédé suivant la revendication 9, qui comprend en outre, après l'étape (c), l'étape de mise en contact du conjugué immobilisé dans des conditions de digestion avec une endonucléase de restriction qui est capable de cliver la portion oligonucléotidique du conjugué de manière à produire un fragment oligomère marqué et un fragment oligomère non marqué ; et dans lequel, dans l'étape (d), les oligomères sont des fragments d'oligomères.

11. Procédé pour séparer un oligonucléotide d'un mélange, ledit procédé comprenant les étapes consistant :
(a) à prendre le conjugué suivant l'une quelconque des revendications 1 à 7, possédant une séquence de nucléotides capable de s'hybrider à une séquence présente à l'intérieur de l'oligonucléotide à séparer ;
(b) à immobiliser le conjugué sur un support ;
(c) à mettre en contact le mélange avec le conjugué immobilisé, dans des conditions provoquant une hybridation de l'oligonucléotide du mélange avec la portion oligonucléotidique du conjugué ; et
(d) à séparer les oligonucléotides hybridés.

12. Procédé pour séparer un oligonucléotide d'un mélange, ledit procédé comprenant les étapes consistant :
(a) à placer le conjugué suivant l'une quelconque des revendications 1 à 7, possédant une séquence de nucléotides capable de s'hybrider à une séquence présente à l'intérieur de l'oligonucléotide à séparer dans une phase hydrophobe ;
(b) à placer le mélange dans une phase hydrophile ;
(c) à mettre en contact la phase contenant le mélange avec la phase contenant le conjugué, dans des conditions provoquant une hybridation de l'oligonucléotide du mélange avec la portion oligonucléotidique du conjugué ; et
(d) à extraire, de la phase contenant le mélange, les oligonucléotides hybridés et à les transporter jusqu'à une phase hydrophile distincte.

13. Liposome comprenant le conjugué suivant l'une quelconque des revendications 1 à 7 et un groupement servant de marqueur.

14. Méthode de détection du lupus érythémateux chez un patient humain, comprenant la mise en contact d'un échantillon de sérum provenant du patient humain avec le liposome suivant la revendication 13, ladite mise en contact étant effectuée de telle sorte que les anticorps présents dans le sérum se lient à l'oligonucléotide présent sur le liposome, de manière à modifier la stabilité du liposome ; et la détermination de la présence ou de l'absence du marqueur.

15. Méthode suivant la revendication 14, dans laquelle les anticorps se lient à l'oligonucléotide sur le liposome de manière à libérer le marqueur, et dans laquelle la quantité du marqueur libéré est mesurée.

16. Utilisation d'un conjugué covalent d'un lipide et d'un oligonucléotide dans la production d'une composition pharmaceutique destinée au traitement d'une maladie associée à la présence d'un acide nucléique pathogène ou d'un oncogène dans les cellules d'un patient.

17. Conjugué covalent d'un lipide et d'un oligonucléotide, ou un de ses sels, destiné à être utilisé comme médicament pour le traitement d'une maladie associée à un acide nucléique pathogène ou oncogène dans les cellules d'un patient, dans lequel l'oligonucléotide possède une séquence de nucléotides suffisamment complémentaire de cet acide nucléique pathogène ou oncogène pour s'hybrider à celui-ci.

18. Conjugué ou sel suivant la revendication 17, qui est conforme à l'une quelconque des revendications 1 à 7.
